# EUROPEAN PATENT APPLICATION

(11) **EP 4 621 055 A1**
(43) Date of publication of application: **24.09.2025**
(21) Application number: 23891441.0
(22) Date of filing: 08.11.2023
(51) Int. Cl.: C12N 15/11, C12M 1/00, C12N 9/88, C12Q 1/686, C12Q 1/6869

(54) **KIT AND METHOD FOR DETECTING DIFFERENCES IN BASE SEQUENCES**

(30) Priority: 15.11.2022 JP 2022182881
(71) Applicant: HIROSAKI UNIVERSITY, Hirosaki-shi, Aomori 036-8560 (JP)
(72) Inventor: FUJITA, Toshitsugu, Hirosaki-shi, Aomori 036-8560 (JP); FUJII, Hodaka, Hirosaki-shi, Aomori 036-8560 (JP)
(74) Representative: Gulde & Partner
(86) International application number: PCT/JP2023/040239
(87) International publication number: WO 2024/106289

(57) **Abstract**

In a DNA-dependent DNA polymerase having 5'-3'exonuclease activity, it was not possible to specifically amplify only mutant DNA having a sequence partially different from the sequence of a target DNA. The purpose of the present invention is to provide a kit comprising a DNA polymerase usable in PCR and a single-stranded RNA, wherein (1) the DNA polymerase is subjected to a temporary inactivation treatment and/or (2) the single-stranded RNA has resistance to degradation by ribonuclease, said DNA polymerase having 5'-3'exonuclease activity, and said single-stranded RNA having a base sequence capable of hybridizing with a target sequence in the target nucleic acid molecule.

## Description

### [Technical Field]

This invention relates to a kit and method for detecting nucleotide sequence differences.

### [Background Art]

Polymerase Chain Reaction (PCR), which is a technique for amplifying specific DNA sequences using a specific pair of primers and DNA-dependent DNA polymerase, is widely used in molecular biology. In PCR reactions, unintended DNA sequences that can also anneal to the primers may be amplified along with the DNA sequence to be amplified. To suppress the amplification of such undesired DNA sequences, a technique known as a blocking PCR method has been developed, which uses blockers-nucleic acids such as DNA and RNA. These blockers bind to complementary sequences within the DNA sequence to be amplified by PCR and inhibit PCR amplification by preventing DNA polymerase extension and primer annealing.

Known blockers to date include long-chain RNA, 3'-modified DNA, artificial nucleic acids, and oligoribonucleotides (ORNs).

Non-Patent Document 1 discloses a method using long-chain RNA (about 750 bases) to specifically inhibit the amplification reaction by PCR.

Non-Patent Documents 2 to 5 disclose methods in which DNA modified at 3' end, artificial nucleic acids, such as locked nucleic acid (LNA) or peptide nucleic acid (PNA), are synthesized to hybridize within the region amplified by PCR and are introduced into the reaction system.

Patent Documents 1 and 2 and Non-Patent Documents 6 and 7 disclose a method (ORNi-PCR method) for using an ORN of about 20-30 bases in length as a blocker.

In addition, Non-Patent Documents 2 and 4 to 7 disclose a method for amplifying only the mutant sequence from a mixture of wild-type and mutant sequences by using blocking PCR utilizing 3'-modified DNA, artificial nucleic acid or ORN as a blocker. Furthermore, Non-Patent Document 5 discloses that when PNA is used as a blocker, a fluorescent dye-labeled probe complementary to the mutant sequence is added to the PCR reaction (using Taq DNA polymerase) at the same time, so that the presence of mutant DNA can be detected in real time by fluorescent emission accompanying the degradation of the probe.

### [RELATED ART DOCUMENTS]

### [PATENT DOCUMETNTS]

[Patent Document 1] JP2016-049107A
[Patent Document 2] WO2019/203350

### [NON-PATENT DOCUMENTS]

[Non-Patent Document 1] Nucleic Acids Res (2001) 29, E31
[Non-Patent Document 12 Nucleic Acids Res (1992) 20, 2493-2496
[Non-Patent Document 3] Biotechniques (1997) 23, 714-716, 718-720
[Non-Patent Document 4] Oncogene (2005) 24, 6830-6834
[Non-Patent Document 5] Cancer Res (2005) 65, 7276-7282
[Non-Patent Document 6] PLoS One, 2014, 9:e113345
[Non-Patent Document 7] DNA Res (2018) 25, 395-407
[Non-Patent Document 8] J Chem Technol Biotechnol (2006) 81, 892-899

### [Disclosure of the Invention]

### [Problems to be Solved by the Invention]

The method disclosed in Non-Patent Document 1 requires the labor-intensive synthesis of long-chain RNA via in vitro transcription. Furthermore, since the amplification of template DNA partially complementary to the long-chain RNA may also be inhibited, the specificity of inhibition is expected to decrease.

When using the methods disclosed in Non-Patent Documents 2 to 4, DNA-dependent DNA polymerases with 3' to 5' exonuclease activity cannot be used because the modification at 3' end may be removed, allowing the inhibitory nucleotide to function as primers. In addition, when using the method disclosed in Non-Patent Document 5, it has been pointed out that PNA may be insoluble in aqueous solution depending on the sequence (Non-Patent Document 8), requiring know-how in the design of the sequence.

The methods disclosed in Patent Documents 1 and 2 and Non-Patent Documents 6 and 7 allow for easy design of ORNs. However, since ORNs may be removed by DNA-dependent DNA polymerases with 5' to 3' exonuclease activity (such as Taq DNA polymerase), it was expected that such polymerases could not be used.

The method disclosed in Non-Patent Document 5 and 7 required expertise in sequence design because Non-Patent Document 8 points out the potential insolubility of PNA in aqueous solutions depending on its sequence.

### [Means of Solving the Problems]

The present inventors have discovered a combination of a DNA polymerase with 5' to 3' exonuclease activity and an ORN that can specifically inhibit the amplification of target DNA containing a sequence complementary to the ORN, while allowing specific amplification of only mutant DNA that differs partially in sequence from the target DNA, in a nucleic acid amplification method using the DNA polymerase having 5'-3' exonuclease activity in combination with the ORN. Based on this discovery, the present invention was completed.

The purpose of the invention is to provide a kit including:
a DNA polymerase usable in PCR; and,
a single-stranded RNA,
in which either (1) the DNA polymerase is temporarily inactivated, or (2) the single-stranded RNA is resistant to degradation by RNA degrading enzymes, or both (1) and (2),
the DNA polymerase has 5' to 3' exonuclease activity, and
the single-stranded RNA has a nucleotide sequence capable of hybridizing to a target sequence in a target nucleic acid molecule.

By using the present kit, even in a nucleic acid amplification method employing a DNA polymerase having 5' to 3' exonuclease activity, the amplification of target DNA having a sequence complementary to the ORN can be specifically inhibited, and only mutant DNA partially differing in sequence from the target DNA can be specifically amplified.

The kit may further include a nucleic acid probe having a fluorescent dye label.

A nucleotide sequence of the nucleotide probe may differ from the nucleotide sequence of the single-stranded RNA by having at least one mutation.

The single-stranded RNA resistant to the degradation by the RNA degrading enzymes may be a phosphorothioate type single-stranded RNA.

The DNA polymerase subjected to the temporary inactivation treatment may be a DNA polymerase modified by a dicarboxylic anhydride.

The kit may further include dUTP.

Another purpose of the invention is to provide a method for specifically inhibiting nucleic acid amplification of a target region, including:
a provision step of providing a mixture including a DNA polymerase usable in PCR, a single-stranded RNA, a target nucleic acid molecule, a pair of DNA primers, and dNTPs;
a denaturation step of maintaining the mixture at a denaturation temperature or higher;
an annealing step of maintaining the mixture at annealing temperature;
a DNA elongation step of maintaining the mixture at a DNA elongation temperature;
an amplification step of amplifying DNA strands by repeating, in order, the denaturation step, the annealing step and the DNA elongation step; and
a detection step of detecting amplification products obtained by the amplification of the DNA strands,
in which either (1) the DNA polymerase is temporarily inactivated, or (2) the single-stranded RNA is resistant to degradation by RNA degrading enzymes, or both (1) and (2),
the DNA polymerase has 5' to 3' exonuclease activity, and
the single-stranded RNA has a nucleotide sequence capable of hybridizing to a target sequence in the target nucleic acid molecule.

Another purpose of the invention is to provide a method for specifically inhibiting nucleic acid amplification of a target region, including:
a provision step of providing a mixture including a DNA polymerase usable in PCR, a single-stranded RNA, a target nucleic acid molecule, a DNA primer, and dNTPs;
a denaturation step of maintaining the mixture at a denaturation temperature or higher;
a maintenance step of maintaining the mixture at a temperature in the range of 45°C to 54°C;
an amplification step of amplifying DNA strands by repeating, in order, the denaturation step and the maintenance step; and
a detection step of detecting amplification products obtained by the amplification of the DNA strands,
in which the DNA polymerase has 5' to 3' exonuclease activity, and
the single-stranded RNA has a nucleotide sequence capable of hybridizing to a target sequence in the target nucleic acid molecule.

By using the present methods, even in a nucleic acid amplification method employing a DNA polymerase having 5' to 3' exonuclease activity, the amplification of target DNA having a sequence complementary to the ORN can be specifically inhibited, and only mutant DNA partially differing in sequence from the target DNA can be specifically amplified.

The mixture may further include a nucleic acid probe having a fluorescent dye label.

A nucleotide sequence of the nucleic acid probe may differ from the nucleotide sequence of the single-stranded RNA by having at least one mutation.

The amplification products may be detected by fluorescence of the fluorescent dye label.

The single-stranded RNA resistant to the degradation by the RNA degrading enzymes may be a phosphorothioate type single-stranded RNA.

The DNA polymerase subjected to the temporary inactivation treatment may be a DNA polymerase modified by a dicarboxylic anhydride.

The dNTPs may include dATP, dUTP, dGTP, and dCTP, and optionally deoxythymidine triphosphate (dTTP) at a concentration lower than the dUTP.

The methods may further include a reverse transcription step of obtaining the target nucleic acid molecule from a target RNA using a reverse transcriptase.

### [Brief Description of the Drawings]

Fig. 1 shows the sequence targeting L858 within the WT EGFR gene (ORN_EGFR_L858), the sequences of WT EGFR corresponding to ORN_EGFR_L858 and the sequences of the L858R mutation thereof (L858R EGFR);
Fig. 2 shows an electrophoresis image of nucleic acids amplified using the THUNDERBIRD Probe qPCR Mix and ORN_EGFR_L858;
Fig. 3 shows an electrophoresis image of nucleic acids amplified using the GoTaq Probe qPCR Master Mix and ORN_EGFR_L858;
Fig. 4 shows an electrophoresis image of nucleic acids amplified using The Premix Taq and ORN_EGFR_L858;
Fig. 5 shows the sequence targeting T790 within the WT EGFR gene (ORN_EGFR_T790_18b), the sequences of WT EGFR corresponding to ORN_EGFR_T790 and the sequence of the T790M mutation thereof (T790M EGFR);
Fig. 6 shows an electrophoresis image of nucleic acids amplified using the THUNDERBIRD Probe qPCR Mix and ORN_EGFR_T790_18b;
Fig. 7 shows an electrophoresis image of nucleic acids amplified using the GoTaq Probe qPCR Master Mix and ORN_EGFR_T790_18b;
Fig. 8 shows an electrophoresis image of nucleic acids amplified using The Premix Taq and ORN_EGFR_T790_18b;
Fig. 9 shows the sequence targeting G13 within the WT KRAS gene (ORN_KRAS_G13), the sequences of WT KRAS corresponding to ORN_KRAS_G13 and the sequence of the G13D mutation thereof (G13D KRAS);
Fig. 10 shows an electrophoresis image of nucleic acids amplified using the THUNDERBIRD Probe qPCR Mix and ORN_KRAS_G13;
Fig. 11 shows an electrophoresis image of nucleic acids amplified using the GoTaq Probe qPCR Master Mix and ORN_KRAS_G13;
Fig. 12 shows an electrophoresis image of nucleic acids amplified using The Premix Taq and ORN_KRAS_G13;
Fig. 13A schematically shows the amplification results using DNA polymerase without 5' to 3' exonuclease activity (α type) and an ORN;
Fig. 13B schematically shows the amplification results using Taq DNA polymerase with 5' to 3' exonuclease activity and an ORN at temperatures near the melting point (Tm) of ORN/DNA hybridization;
Fig. 13C schematically shows the amplification results using Taq DNA polymerase with 5' to 3' exonuclease activity and an ORN at temperatures below the melting point (Tm) of ORN/DNA hybridization;
Fig. 14A shows an electrophoresis image of nucleic acids amplified using GeneAce Taq and ORN_EGFR_L858;
Fig. 14B shows the results of DNA sequence analysis of the amplification products indicated by an arrow in Fig. 14A;
Fig. 15 schematically shows the suppression mode for nucleic acid amplification in ORNi-PCR with GeneAce Taq at temperature near the Tm of ORN/DNA hybridization;
Fig. 16 shows an electrophoresis image of nucleic acids amplified using GeneAce Taq and ORN_EGFR_T790_18b;
Fig. 17 shows an electrophoresis image of nucleic acids amplified using GeneAce Taq and ORN_KRAS_G13;
Fig. 18 shows the nucleotide sequence of the EGFR gene amplified using the forward primer (hEGFR-Exon21-F8) and the reverse primer (hEGFR-Exon21-R8);
Fig. 19 shows the sequence targeting L858 within the WT EGFR gene (ORN_EGFR_L858_17b), the sequences of WT EGFR corresponding to ORN_EGFR_L858_17b and the sequences of the L858R mutation thereof (L858R EGFR);
Fig. 20A shows an electrophoresis image of nucleic acids amplified using GeneAce Taq and ORN_EGFR_L858_17b;
Fig. 20B shows the results of DNA sequence analysis of the amplification products indicated by an arrow in Fig. 20A;
Fig. 21 shows an electrophoresis image of nucleic acids amplified using ORN_EGFR_L858 (during the annealing and elongation steps at 56°C);
Fig. 22 shows an electrophoresis image of nucleic acids amplified using ORN_EGFR_L858 (during the annealing and elongation steps at 59°C);
Fig. 23 shows a schematic diagram of conventional real-time PCR using a double-labeled fluorescent probe;
Fig. 24 shows a schematic diagram of real-time ORNi-PCR using a double-labeled fluorescent probe;
Fig. 25 shows the sequences of various double-labeled fluorescent probes targeting the L858R EGFR mutation;
Fig. 26 shows the results regarding conventional real-time PCR using Probe_EGFR_L858R_15b_2;
Fig. 27 shows the results regarding conventional real-time PCR using Probe_EGFR_L858R_16b;
Fig. 28 shows the results regarding conventional real-time PCR using Probe_EGFR_L858R_18b;
Fig. 29 shows the results regarding conventional real-time PCR using Probe_EGFR_L858R_Sense_15b;
Fig. 30A shows the results regarding real-time ORNi-PCR using ORN_EGFR_L858_17b and Probe_EGFR_L858R_18b in the case where the L858R EGFR gene is 1% of the total EGFR template;
Fig. 30B shows the results of DNA sequence analysis of the amplification products.
Fig. 31A shows the results regarding real-time ORNi-PCR using ORN_EGFR_L858_17b and Probe_EGFR_L858R_18b in the case where the L858R EGFR gene is 0.2% of the total EGFR template;
Fig. 31B shows the results of DNA sequence analysis of the amplification products;
Fig. 32A shows the results regarding conventional real-time PCR using Probe_EGFR_L858R_18b in the case where the L858R EGFR gene is 1% of the total EGFR template;
Fig. 32B shows the results of DNA sequence analysis of the amplification products;
Fig. 33A shows the results regarding real-time ORNi-PCR using ORN_EGFR_L858 and Probe_EGFR_L858R_Sense_15b in the case where the L858R EGFR gene is 1% of the total EGFR template;
Fig. 33B shows the results of DNA sequence analysis of the amplification products.
Fig. 34A shows the results regarding conventional real-time PCR using Probe_EGFR_L858R_Sense_15b in the case where the L858R EGFR gene is 1% of the total EGFR template;
Fig. 34B shows the results of DNA sequence analysis of the amplification products;
Fig. 35A shows the results regarding real-time ORNi-PCR using Probe_EGFR_L858R_18b and ORN_EGFR_L858_17b in the case where the L858R EGFR gene is 5% of the total EGFR template;
Fig. 35B shows the results of DNA sequence analysis of the amplification products;
Fig. 36A shows the results regarding conventional real-time PCR using Probe_EGFR_L858R_18b in the case where the L858R EGFR gene is 5% of the total EGFR template;
Fig. 36B shows the results of DNA sequence analysis of the amplification products;
Fig. 37 shows an electrophoresis image of nucleic acids amplified using the Hot-Start Gene Taq NT and ORN_EGFR_L858_17b in the presence of dTTP or dUTP;
Fig. 38 shows an electrophoresis image of nucleic acids amplified using the Hot-Start Gene Taq NT, ORN_EGFR_L858_17b and 293T cell-derived genomic DNA or NCI-H1975 cell-derived genomic DNA in the presence of dUTP;
Fig. 39 shows the results of DNA sequence analysis of the amplification products indicated by an arrow in Fig. 38;
Fig. 40A shows an electrophoresis image of nucleic acids amplified using AmpliTaq Gold 360, ORN_EGFR_L858_17b and 293T cell-derived genomic DNA or NCI-H1975 cell-derived genomic DNA in the presence of dTTP or dUTP;
Fig. 40B shows the results of DNA sequence analysis of the amplification products indicated by an arrow in Fig. 40A;
Fig. 41 shows an electrophoresis image of nucleic acids amplified using unmodified Taq DNA polymerase (Gene Taq NT) and ORN_EGFR_L858_17b in the presence of dUTP;
Fig. 42 shows an electrophoresis image of nucleic acids amplified using unmodified Taq DNA polymerase (Gene Taq NT), ORN_EGFR_L858_17b and the Hot-Start Gene Taq NT reaction buffer in the presence of dUTP;
Fig. 43 shows an electrophoresis image of nucleic acids amplified using unmodified Taq DNA polymerase (Gene Taq NT) and ORN_EGFR_L858_17b in the presence of dUTP and under a long heat treatment condition;
Fig. 44A shows electrophoresis images of nucleic acids amplified using conventional Taq DNA polymerase (THUNDERBIRD Probe qPCR Mix), ORN_EGFR_L858_S_All and 293T cell-derived genomic DNA or NCI-H1975 cell-derived genomic DNA;
Fig. 44B shows the results of DNA sequence analysis of the amplification products indicated by an arrow in Fig. 44A;
Fig. 45 shows an electrophoresis image of nucleic acids amplified using conventional Taq DNA polymerase (THUNDERBIRD Probe qPCR Mix), ORN (ORN_EGFR_L858_S_5) partially modified with five phosphodiester bonds at the 5' end;
Fig. 46 shows an electrophoresis image of nucleic acids amplified using conventional Taq DNA polymerase (GoTaq Probe qPCR Master Mix), fully phosphorothioated ORN (ORN_EGFR_L858_S_All) and 293T cell-derived genomic DNA or NCI-H1975 cell-derived genomic DNA;
Fig. 47A shows an electrophoresis image of nucleic acids amplified using conventional Taq DNA polymerase (THUNDERBIRD Probe qPCR Mix), fully phosphorothioated ORN (ORN_EGFR_L858_S_All) and 293T cell-derived genomic DNA or NCI-H1975 cell-derived genomic DNA;
Fig. 47B shows the results of DNA sequence analysis of the amplification products indicated by an arrow in Fig. 47A;
Fig. 48 shows an electrophoresis image of nucleic acids amplified using conventional Taq DNA polymerase (THUNDERBIRD Probe qPCR Mix) and fully phosphorothioated ORN (ORN_EGFR_L858_S_All) at 30 seconds or 2 minutes for the annealing and extension steps;
Fig. 49A shows the results regarding real-time ORNi-PCR using ORN_EGFR_L858_S_All and Probe_EGFR_L858R_Sense_15b;
Fig. 49B shows the results of DNA sequence analysis of the amplification products;
Fig. 50A shows the results regarding conventional real-time PCR using Probe_EGFR_L858R_Sense_15b;
Fig. 50B shows the results of DNA sequence analysis of the amplification products;
Fig. 51A shows electrophoresis images of nucleic acids amplified using 0.1, 0.2 and 1 µM fully phosphorothioated ORN (ORN_EGFR_L858_S_All);
Fig. 51B shows the results of DNA sequence analysis of the amplification products indicated by an arrow in Fig. 51A.
Fig. 52 shows an electrophoresis image of nucleic acids amplified using ORN (ORN_EGFR_L858_S_5) partially modified with five phosphodiester bonds at the 5' end;
Fig. 53A shows the results regarding real-time ORNi-PCR using ORN_EGFR_L858_S_All and Probe_EGFR_L858R_Sense_15b in the case where the L858R EGFR mutation represents 1% of the total EGFR template;
Fig. 53B shows the results of DNA sequence analysis of the amplification products;
Fig. 54 schematically shows the scheme of the one-step RT-ORNi-PCR;
Fig. 55 schematically shows the location of a mutation on the WT EGFR gene, ORN, probe and pair of primers;
Fig. 56A shows an electrophoresis image of nucleic acids amplified by one-step RT-ORNi-PCR with ORN_EGFR_L858_S_All (0.5 µM ORNs; during the annealing and elongation steps at 57.5°C) detecting the L858R EGFR mutation, using total RNA extracted from each of NCI-H1975 cells and MRC-5 cells with WT EGFR;
Fig. 56B shows the results of DNA sequence analysis of the amplification products indicated by an arrow in Fig. 56A.
Fig. 57 shows electrophoresis images of nucleic acids amplified by one-step RT-ORNi-PCR with ORN_EGFR_L858_S_All (0.25, 0.5 and 1 µM ORNs; during the annealing and elongation steps at 56 or 59°C) detecting L858R EGFR mutation, using total RNA extracted from each of MRC-5 cells and NCI-H1975 cells;
Fig. 58 shows an electrophoresis image of nucleic acids amplified by one-step RT-ORNi-PCR with unmodified ORN_EGFR_L858 (0.5 and 6 µM ORNs) using total RNA extracted from each of MRC-5 cells and NCI-H1975 cells;
Fig. 59 schematically shows the scheme of one-step real-time RT-ORNi-PCR;
Fig. 60A shows the results of one-step real-time RT-PCR using the QIAGEN OneStep RT-PCR Kit and Probe_EGFR_L858R_Sense_15b;
Fig. 60B shows the results of DNA sequence analysis of the amplification products;
Fig. 61A shows the results regarding one-step real-time RT-ORNi-PCR using the QIAGEN OneStep RT-PCR Kit, Probe_EGFR_L858R_Sense_15b and ORN_EGFR_L858_S_All;
Fig. 61B shows the results of DNA sequence analysis of the amplification products;
Fig. 62 schematically shows the scheme of one-step real-time RT-ORNi-PCR using the iTaq Universal Probes One-Step Kit;
Fig. 63A shows the results regarding one-step real-time RT-PCR using Probe_EGFR_L858R_Sense_15b and the iTaq Universal Probes One-Step Kit containing unmodified Taq DNA polymerase;
Fig. 63B shows the results of DNA sequence analysis of the amplification products;
Fig. 64A shows the results regarding one-step real-time RT-ORNi-PCR using the iTaq Universal Probes One-Step Kit, Probe_EGFR_L858R_Sense_15b and ORN_EGFR_L858_S_All;
Fig. 64B shows the results of DNA sequence analysis of the amplification products;
Fig. 65A shows an electrophoresis image of nucleic acids amplified by one-step RT-ORNi-PCR with ORN_EGFR_L858_S_All (0.25 and 0.5 µM ORNs) using total RNA extracted from MRC-5 cells and NCI-H1975 cells;
Fig. 65B shows the results of DNA sequence analysis of the amplification products indicated by an arrow in Fig. 65A;
Fig. 66 schematically illustrates the experimental steps of a one-step RT-ORNi-PCR reaction designed to evaluate the sequence-specific inhibition of DNA extension by ORN_EGFR_L858R_S_All;
Fig. 67A shows an image of electrophoresis results indicating that ORN_EGFR_L858_S_All specifically inhibited the amplification of target DNA from cDNA in a sequence-specific manner during the PCR step;
Fig. 67B shows the results of DNA sequence analysis of the amplification products indicated by an arrow in Fig. 67A;
Fig. 68A shows an image of the electrophoresis results indicating that DNA amplification was non-specifically inhibited when PCR was performed after RT in the presence of ORN_EGFR_L858_S_All;
Fig. 68B shows the results of DNA sequence analysis of the amplification products indicated by an arrow in Fig. 68A;
Fig. 69 schematically illustrates the hybridization site of a phosphorothioated ORN that is non-complementary to EGFR mRNA;
Fig. 70 shows the nucleotide sequence of ORN_EGFR_L858_Sense_S_All that is a phosphorothioated ORN non-complementary to EGFR mRNA, and the corresponding sequences of WT EGFR mRNA and L858R EGFR mRNA;
Fig. 71A shows an electrophoresis image of nucleic acids amplified by one-step RT-ORNi-PCR with ORN_EGFR_L858_Sense_S_All (0.1 and 0.25 µM ORNs) using total RNA extracted from MRC-5 cells and NCI-H1975 cells;
Fig. 71B shows the results of DNA sequence analysis of the amplification products indicated by an arrow in Fig. 71A;
Fig. 72A shows an image of electrophoresis results indicating that ORN_EGFR_L858_Sense_S_All specifically inhibited the amplification of target DNA from cDNA in a sequence-specific manner during the PCR step;
Fig. 72B shows the results of DNA sequence analysis of the amplification products indicated by an arrow in Fig. 72A;
Fig. 73A shows an image of electrophoresis results indicating that DNA amplification was non-specifically inhibited in the case where PCR was performed after RT in the presence of ORN_EGFR_L858_Sense_S_All; and
Fig. 73B shows the results of DNA sequence analysis of the amplification products indicated by an arrow in Fig. 73A.

### [Description of Embodiments]

### Definition

For convenience, certain terms employed in the context of the present disclosure are collected here. Unless defined otherwise, all technical and scientific terms used herein have the same meaning as commonly understood by one of the ordinary skilled in the art to which this invention belongs. The singular forms "a", "an", and "the" are used herein to include plural referents unless the context clearly dictates otherwise.

Notwithstanding that the numerical ranges and parameters setting forth the broad scope of the invention are approximations, the numerical values set forth in the specific examples are described as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art.

Hereinafter, embodiments of the present invention are illustrated in detail. The following embodiments are illustrative only and do not limit the scope of the present invention. In order to avoid redundancy, explanation for similar contents is not repeated.

### 1. Kit

The kit according to the present embodiment includes a DNA polymerase usable in PCR and a single-stranded RNA.

### DNA Polymerase

A DNA polymerase functions as an enzyme responsible for DNA synthesis within cells. In vivo, the DNA polymerases are involved in DNA synthesis processes such as DNA replication, DNA repair, recombination, and gene amplification. A DNA polymerase usable in PCR refers to a DNA polymerase whose activity is not lost even when exposed to the temperatures to be set in PCR, and is also referred to as a heat-resistant DNA polymerase. The DNA polymerase in the present embodiment has 5' to 3' exonuclease activity. The 5' to 3' exonuclease activity refers to the ability to sequentially remove nucleotides from the 5' end of an oligonucleotide.

In one embodiment, the DNA polymerase is subjected to temporary inactivation treatment. The DNA polymerase that has been subjected to temporary inactivation treatment exhibits DNA polymerase activity upon reaching a specific ambient environment (e.g., temperature). Temporary inactivation treatments include wax barriers (U.S. Patent Nos. 5,599,660 and 5,411,876), antibodies and chemical modifications that temporarily inactivate DNA polymerase. In one embodiment, the chemical modification is a dicarboxylic anhydride modification. The DNA polymerase with temporary inactivation treatment may be a DNA polymerase modified with dicarboxylic anhydride or a DNA polymerase to which antibodies that temporarily inactivate the DNA polymerase are added. In one embodiment, dicarboxylic anhydrides include citraconic anhydride, 2-methyl maleic anhydride, 2,3-dimethyl maleic anhydride, cis-aconitic anhydride and tetrafluorosuccinic anhydride.

In one embodiment, the DNA polymerase is a DNA polymerase capable of hot-start PCR. The hot-start PCR is a PCR method that isolates the DNA polymerase from the sample DNA until a specific temperature is reached.

### Single-Stranded RNA

A single-stranded RNA has a nucleotide sequence hybridizable to a target sequence (target region) in a target nucleic acid molecule. "Hybridizable" means that the nucleic acid molecule and the single-stranded nucleic acid can be complementarily bound in nucleic acid amplification methods such as PCR.

The single-stranded RNA according to the present embodiment can specifically inhibit nucleic acid amplification of the target region in the target nucleic acid molecule by complementary binding of the single-stranded RNA to the target nucleic acid molecule in the target sequence of the target nucleic acid molecule.

In one embodiment, the single-stranded RNA is hybridizable to the target sequence (target region) on one allele and does not hybridize to the mutant sequence (mutant region) corresponding to the target sequence (target region) on the other allele except under specific temperature conditions. Therefore, the mutant sequence (mutant region) can be rephrased as a region where single-stranded RNA hybridizes under specific temperature conditions. The target sequence differs from the mutant sequence by having at least one mutation. Thus, the nucleic acid molecule according to this embodiment includes the target nucleic acid molecule. In one embodiment, the nucleic acid molecule includes a target nucleic acid molecule and a mutant nucleic acid molecule having a mutant sequence (mutant region).

The conditions of the specific temperature are around the Tm value of the single-stranded RNA, and "around the Tm value" refers to Tm ±1°C, ±2°C, ±3°C, or ±4°C.

The Tm value can be calculated based on known calculation methods such as the nearest neighbor base pair method and GC% method. However, it is more preferable to calculate the Tm value using the following formula: Tm = (a + u) * 2 + (g + c) * 4, where a, u, g, and c indicate the number of bases in A, U, G, and C, respectively.

In one embodiment, if the single-stranded RNA is also to hybridize to a mutant sequence (mutant region) on the other allele, the single-stranded RNA and the target nucleic acid molecule can be hybridized under temperatures lower than the Tm value of the single-stranded RNA (for example, a temperature 1°C and 8°C lower than the Tm value, or a range between two values selected from the group consisting of 1°C, 2°C, 3°C, 4°C, 5°C, 6°C, 7°C and 8°C lower than the Tm value). In another embodiment, if the single-stranded RNA is also to hybridize to the mutant sequence (mutant region) on the other allele, the single-stranded RNA and the target nucleic acid molecule can be hybridized in a range of 45°C to 56°C (for example, a range between two values selected from the group consisting of 45°C, 46°C, 47°C, 48°C, 49°C, 50°C, 51°C, 52°C, 53°C, 54°C, 55°C and 56°C).

In one embodiment, the single-stranded RNA is resistant to degradation by RNA degrading enzymes (such as ribonucleases). High resistance of the RNA to degradation by the RNA degrading enzymes can stabilize the antisense oligonucleotide. In one embodiment, the single-stranded RNA resistant to degradation by the RNA degrading enzymes is a phosphorothioate type single-stranded RNA. A phosphorothioate type RNA means RNA in which the oxygen atom of the phosphate group of an oligonucleotide having a phosphodiester bond is replaced by a sulfur atom. The phosphorothioate type single-stranded RNA has phosphorothioate modifications on some or all phosphodiester bonds. Some phosphodiester bonds have phosphorothioate modifications in an amount of 60% or more, 70% or more, 80% or more, 90% or more, 95% or more, 98% or more, or 99% or more and 100% or less of the phosphodiester bonds.

In the kit according to the present embodiment, (i) the DNA polymerase is chemically modified, (ii) the single-stranded RNA is resistant to degradation by RNA degrading enzymes, or (iii) the DNA polymerase is chemically modified and the single-stranded RNA is resistant to degradation by RNA degrading enzymes.

To design single-stranded RNA (single-stranded nucleic acid) that hybridizes to the target region, nucleotide sequence information of the target region is necessary. If the nucleotide sequence of the template nucleic acid (target nucleic acid) and the location of the target region within the template nucleic acid are known, the nucleotide sequence information of the target region can be obtained based on them. If the nucleotide sequence information of the target region is unknown, the amplification products other than the target amplified by the nucleic acid amplification reaction can be isolated by a known method such as agarose gel electrophoresis, and subjected to a known sequencing method to obtain the nucleotide sequence information of the target region. Based on the obtained sequence information of the target region, a single-stranded nucleic acid that hybridizes to the target region can be designed. If the template nucleic acid is double-stranded (e.g., a double-stranded DNA consisting of sense and antisense strands), the single-stranded nucleic acid can hybridize to either strand.

It is preferable that the single-stranded RNA (single-stranded nucleic acid) be completely complementary to the nucleotide sequence of the target region. However completed complementarity is not required as long as the nucleic acid amplification of the target region can be specifically inhibited. As long as hybridization to the target region is possible, one or several mismatches (for example, two, three, or four) may be allowed. The single-stranded nucleic acids that hybridize to the target region include single-stranded nucleic acids that are complementary to 80% or more of the target sequence, preferably 90% or more of the target sequence, more preferably 95% or more of the target sequence, and even more preferably 98% or more of the target sequence, and that can specifically inhibit nucleic acid amplification of the target region.

The single-stranded nucleic acid that hybridizes to the target region may be RNA or a chimera of RNA and another nucleic acid, but is preferably single-stranded RNA. Other nucleic acids include DNA, modified DNA, and modified RNA. When the single-stranded nucleic acid is a chimera of RNA and another nucleic acid, the other nucleic acid preferably constitutes 50% or less of the total nucleotide length, more preferably 40% or less of the total nucleotide length, even more preferably 30% or less of the total nucleotide length, even more preferably 20% or less of the total nucleotide length, even more preferably 10% or less, and even more preferably 5% or less of the total nucleotide length.

The length (base length) of the single-stranded nucleic acid that hybridizes to the target region is not particularly limited, but it is preferably 10 to 200 bases, more preferably 10 to 150 bases, more preferably 10 to 120 bases, more preferably 10 to 100 bases, more preferably 10 to 90 bases, even more preferably 10 to 80 bases, even more preferably 10 to 70 bases, even more preferably 10 to 60 bases, even more preferably 10 to 50 bases, even more preferably 12 to 45 bases, even more preferably 14 to 40 bases, even more preferably 16 to 35 bases, even more preferably 18 to 32 bases, even more preferably 20 to 30 bases, even more preferably 21 to 28 bases and even more preferably 22 to 26 bases. A particularly preferred length is 23 bases.

The single-stranded nucleic acids that hybridize to the target region may have modifications at the 5' end and/or the 3' end. For example, the 5' end and/or the 3' end may be modified by phosphorylation, amination, biotinylation, thiolation, cholesterolation, DIG (digoxigenin) labeling, quencher modification such as BHQ-1 and BHQ-3, fluorescent modification such as DNP, Cy3, Cy5, TAMRA and 6-FAM can be used.

The single-stranded nucleic acids that hybridize to the target region may contain nucleotides (ribonucleotides or deoxyribonucleotides) in which the sugar, base, and/or phosphate group are chemically modified, as long as hybridization to the target region is possible. Base-modified nucleotides include, for example, 5-position-modified uridine or cytidine (e.g., 5-propynyluridine, 5-propynylcytidine, 5-methylcytidine, 5-methyluridine, 5-(2-amino)propyluridine, 5-halocytidine, 5-halouridine, and 5 methyloxyuridine); 8-position modified adenosine or guanosine (e.g., 8-bromogunosine); deaza-nucleotides (e.g., 7-deaza-adenosine); O- and N-alkylated nucleotides (e.g., N 6-methyladenosine). Furthermore, sugar-modified nucleotides include 2'-position sugar modifications where the 2'-OH of a ribonucleotide is substituted with, for example, H, OR, R, a halogen atom, SH, SR, NH2, NHR, NR2, or CN (where R represents an alkyl group, alkenyl group, or alkynyl group with 1 to 6 carbon atoms), and 5'-end phosphorylation modifications where the 5' end is monophosphorylated. Phosphate-modified nucleotides include a nucleotide in which the phosphoester group linking adjacent ribonucleotides is replaced with a phosphothioate group.

The single-stranded nucleic acids that hybridize to the target region can be produced by artificial chemical synthesis using known methods. They can also be produced from template DNA via in vitro transcription.

### Nucleic Acid Probe with Fluorescent Dye Label

The kit according to the present embodiment may further include a nucleic acid probe with a fluorescent dye label. The nucleic acid probe with the fluorescent dye label according to the present is used in the real-time PCR method, especially in the probe method. The nucleic acid probe with the fluorescent dye label used in the probe method is equipped with a fluorescent substance (such as FAM) at the 5' end and a quencher substance (such as TAMRA) at the 3' end, or a fluorescent substance at the 3' end and a quencher substance at the 5' end. The nucleic acid probe is also referred to herein simply as a probe. The nucleic acid probe may be a single-stranded DNA probe.

The nucleotide sequence of the nucleic acid probe differs from the above sequence of the above single-stranded RNA by having at least one mutation. In one embodiment, the nucleic acid probe hybridizes to a mutant sequence (mutant region) on an allele different from the allele to which the single-stranded RNA hybridizes.
Thus, the single-stranded RNA hybridizes to the target sequence (target region) on one allele, and the nucleic acid probe hybridizes to the mutant sequence (mutant region) on the other allele.
Thus, the nucleic acid probe can hybridize complementarily to the mutant sequence in the target nucleic acid molecule.

The kit according to the present embodiment may further include dUTP. The kit according to the present embodiment may further include a pair of primers.

### 2. Method for Specifically Inhibiting Nucleic Acid Amplification of Target Regions

A method for specifically inhibiting nucleic acid amplification of a target region according to the present embodiment has a provision step, a denaturation process, an annealing step, a DNA elongation step, an amplification step, and a detection step. The method can be performed in the same reaction system. Furthermore, an additional nucleic acid amplification step may be performed after this method.

### 2-1. Provision Step

In the provision step, a mixture containing a DNA polymerase usable in PCR, a single-stranded RNA, a target nucleic acid molecule, a pair of DNA primers, and dNTPs is provided.

The concentration of the single-stranded RNAin the mixture is not limited as long as it is a concentration that can specifically inhibit nucleic acid amplification of the target region. It is preferable to conduct a preliminary testing for each specific condition of the nucleic acid amplification reaction to be applied, and to set the concentration as appropriate. Specifically, for example, 10 µM or less is preferred, 5 µM or less is more preferred, 1 µM or less is even more preferred, 500 nM or less is even more preferred, 200 nM or less is even more preferred, 100 nM or less is even more preferred, 90 nM or less is even more preferred, 80 nM or less is even more preferred, 70 nM or less is even more preferred, 60 nM or less is even more preferred, 50 nM or less is even more preferred. The lower limit is not particularly limited, but 10 nM or more is preferred, 20 nM or more is more preferred, 30 nM or more is even more preferred, and 40 nM or more is even more preferred.

In addition to the DNA polymerase, single-stranded RNA, nucleic acid molecule, pair of DNA primers, and dNTP, buffer, and salt are added to the mixture. If necessary, an enzyme protectant, a melting temperature (Tm) adjuster, a surfactant, and other agents may be added. The buffer such as Tris-HCl is used to buffer the mixture from neutral to slightly alkaline. pH is adjusted to near the optimum pH depending on the DNA polymerase to be used. The salt is added as needed to maintain enzyme activity and adjust the melting temperature (Tm) of nucleic acids, specifically KCl, NaCl, MgCl₂, MgSO₄, or (NH₄)₂SO₄ is used. Bovine serum albumin or sugar is used as enzyme protectants. In addition, dimethyl sulfoxide (DMSO), formamide, or betaine (N,N,N, -trimethylglycine) is used as the melting temperature (Tm) adjuster. Tween 20 or TritonX is used as the surfactant.

The pair of DNA primers can amplify the first amplified region including the target sequence (target region) and the second amplified region including the mutant sequence (mutant region) in the target nucleic acid molecule in the nucleic acid amplification method. In other words, the pair of DNA primers includes a forward primer and a reverse primer, in which the forward primer is designed to be upstream of the first and second amplified regions and the reverse primer is designed to be downstream of the first and second amplified regions.

The dNTP includes deoxyadenosine triphosphate (dATP), deoxyuridine triphosphate (dUTP), deoxyguanosine triphosphate (dGTP), deoxycytidine triphosphate (dCTP), and optionally deoxythymidine triphosphate (dTTP) at lower concentrations than dUTP.

In one embodiment, the dNTP does not contain the dTTP. In another embodiment, the dNTP contains the dTTP and the concentration of dTTP is lower than the concentration of dUTP. The dTTP may be 10% or less, 5% or less, 4% or less, 3% or less, 2% or less, 1% or less, 0.5% or less, 0.1% or less, 0.01% or less of the concentration of dUTP.

### 2-2. Denaturation Step

In the denaturation step, the mixture is maintained at or above denaturation temperature. The denaturation temperature is temperature at which double-stranded nucleic acids (nucleic acid molecules) are reversibly thermally denatured (diverged) to single-stranded nucleic acids and is generally 90°C or higher, 91°C or higher, 92°C or higher, 93°C or higher, 94°C or higher, 95°C or higher, or 98°C or higher. The denaturation step is performed for 10 to 60 seconds, but is not limited thereto.

### 2-3. Annealing Step

In the annealing step, the mixture is maintained at annealing temperature. In the annealing step, the single-stranded RNA and pair of primers bind to the nucleic acid molecule (template DNA), which has been rendered single-stranded by heat denaturation, to form double-stranded structures (a priming reaction). The annealing temperature may be a range between 50°C and 72°C or a range between two values selected from the group consisting of 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C and 72°C. Annealing time is typically 10 to 30 seconds, but is not limited thereto.

.

### 2-4. DNA elongation Step

In the DNA elongation step, the mixture is maintained at DNA elongation temperature. In the DNA elongation step, DNA is elongated by DNA polymerase starting from the primer. The DNA elongation temperature varies with the DNA polymerase, typically around 72°C. The DNA elongation step can be modified depending on the desired amplified strand length, typically, but not limited to, 1 kb amplified strand length/1 minute.

The temperatures for the annealing and DNA elongation steps may be the same.

### 2-5. Amplification Step

In the amplification step, the DNA strands are amplified by repeating the denaturation step, annealing step and DNA elongation step in order. The number of cycles of the amplification step is typically 25 to 40, but is not limited thereto.

### 2-6. Detection Step

In the detection step, amplification products obtained by amplification of the DNA strand are detected. Specific inhibition of nucleic acid amplification of the target region can be confirmed, for example, by the amount of amplification products of the target region in the reaction mixture after the nucleic acid amplification reaction. The amount of amplification products of the target region in the mixture after the nucleic acid amplification reaction can be confirmed, for example, by subjecting the reaction solution after the nucleic acid amplification reaction to agarose gel electrophoresis and checking the concentration of the band of amplification products of the target region. If no band of amplification products of the target region are detected, or if the amount of amplification products of the target region is reduced compared to the case where no single-stranded nucleic acid is added to the reaction system, it can be judged that the nucleic acid amplification of the target region is specifically inhibited. Therefore, when performing the nucleic acid amplification suppression method of the present invention, it is preferable to use a reaction system to which the single-stranded nucleic acid that hybridizes to the target region is added as well as a reaction system to which no such single-stranded nucleic acid is added as a control. The amplification products can be separated by agarose gel electrophoresis or other known methods, and the nucleotide sequence information of the amplification products can be obtained by subjecting it to known sequencing analysis methods.

When the amplification products are detected by real-time PCR, the mixture has the nucleic acid probe with the fluorescent dye label. In this case, the amplification products are detected by fluorescence from the fluorescent dye label.

### 2-6. Reverse Transcription Step

In the reverse transcription step, the nucleic acid molecule is obtained from a target RNA using a reverse transcriptase. The reverse transcription step is effective when a mutation in RNAis to be detected, as converting the RNAinto cDNA allows the use of this method.

### 3. Method for Specifically Inhibiting Nucleic Acid Amplification of Target Region

The method for specifically inhibiting nucleic acid amplification of a target region according to the present embodiment includes a provision step, a denaturation process, a maintenance step, an amplification step and a detection step.

### 3-1. Maintenance Step

In the maintenance step, the mixture is maintained at 45°C to 54°C.

### [EXAMPLES]

### Experimental Materials and Methods

### Oligonucleotide

ODN (oligodeoxyribonucleotide) was chemically synthesized at Eurofins Genomics K.K. (Tokyo, Japan) and used as primers. Double-labeled fluorescent ODN was chemically synthesized at Fasmac Co., Ltd. (Tokyo, Japan). ORN (oligoribonucleotide) and phosphorothioate-modified ORN were chemically synthesized and purified by high performance liquid chromatography (Fasmac Co., Ltd.).

### Template DNA and RNA

Genomic (g)DNA was extracted from 293T, NCI-H1975 and HCT116 cells using standard phenol-chloroform extraction. 293T cells have wild-type (WT) epidermal growth factor receptor (EGFR) and KRAS genes. NCI-H1975 cells have a mutant EGFR sequence corresponding to T790M and L858R on one allele. HCT116 cells have a mutant KRAS sequence corresponding to G13D on one allele. A 10 ng gDNA mixture including WT and mutant EGFR genes (with mutant-to-WT ratios ranging from 5% to 0.2%) was prepared by mixing gDNA from 293T and NCI-H1975 cells. The copy number of single-copy genes can be calculated from the concentration of gDNA used as template and the average mass of DNA in the cell. In general, 10 ng of template gDNA contains approximately 3,333 copies of a single-copy gene (e.g., EGFR); therefore, a mutant-to-WT ratio of 1% corresponds to the presence of 33 copies of the gene. From FFPE samples including human cells carrying either WT EGFR (HD141, Horizon Discovery, Cambridge, UK) or both WT and mutant EGFR genes (HD300, Horizon Discovery), DNA was extracted using the Quick-DNA FFPE Kit (Zymo Research, Irvine, CA, USA). RNA was extracted from MRC-5 and NCI-H1975 cells using Isogen II (Nippon Gene, Tokyo, Japan). MRC-5 cells have WT EGFR gene sequence.

### ORNi-PCR and Real-Time ORNi-PCR with Taq DNA Polymerase

ORNi-PCR was performed using 10 ng of gDNA and one of the following reagents: the THUNDERBIRD Probe qPCR Mix (TOYOBO, Osaka, Japan), the GoTaq Probe qPCR Master Mix (Promega, Madison, WI, USA), the Premix Taq (TaKaRa Taq Version) (Takara Bio, Shiga, Japan), the GeneAce Probe qPCR Mix II (Nippon Gene, Tokyo, Japan), or the EmeraldAmp MAX PCR Master Mix (Takara Bio). A reaction mixture (10 µL) was prepared according to the manufacturers' protocols and subjected to ORNi-PCR using either the Mastercycler nexus (Eppendorf, Hamburg, Germany) or the LifeEco ver. 2.0 (Hangzhou Bioer Technologies, Hangzhou, China).

In real-time ORNi-PCR, a dual-labeled fluorescent probe (0.2 µM) was added to the reaction mixture, which was then subjected to the real-time ORNi-PCR using the CFX Connect Real-Time PCR Detection System (Bio-Rad Laboratories, Hercules, CA, USA). ORNi-PCR products were electrophoresed on a 2 to 3% agarose gel. When necessary, each product was subjected to DNA sequencing (Eurofins Genomics). DNA sequence analysis was performed using the Applied Biosystems Sequence Scanner Software v2.0 (Thermo Fisher Scientific, Waltham, MA, USA).

### ORN-Mediated Blocking Test Using Chemically Modified Taq DNA Polymerase

ORNi-PCR was performed using 10 ng of gDNA and either the Hot-Start Gene Taq NT containing chemically modified the Taq DNA polymerase (Nippon Gene), the AmpliTaq Gold 360 DNA Polymerase (Thermo Fisher Scientific, Vilnius, Lithuania), or Gene Taq NT containing unmodified (conventional) Taq DNA polymerase (Nippon Gene). The Hot-Start Gene Taq NT contains the same Taq DNA polymerase used in the GeneAce Probe qPCR Mix II (Nippon Gene). Reaction mixtures (10 µL) were prepared using a dNTP mix containing either dTTP (a component of the kit) or dUTP (NTP-501, TOYOBO), which was then subjected to ORNi-PCR using the Mastercycler nexus (Eppendorf) or the LifeEco ver. 2.0 (Hangzhou Bioer Technologies).

### One-Step RT-ORNi-PCR and One-Step Real-Time RT-ORNi-PCR

One-step RT-ORNi-PCR was performed using 25 ng of RNA and the QIAGEN OneStep RT-PCR Kit (Qiagen, Hilden, Germany). dNTP mixture containing dUTP (TOYOBO) instead of dTTP (a component of the kit) was used to prepare reaction mixture (10 µL), which was then subjected to the one-step RT-ORNi-PCR using the LifeEco ver. 2.0 (Hangzhou Bioer Technologies). Alternatively, the one-step RT-ORNi-PCR was performed using 25 ng of RNA and the iTaq Universal Probes One-Step Kit (Bio-Rad Laboratories). Reaction mixtures (10 µL) were prepared according to the manufacturer's protocol and subjected to the one-step RT-ORNi-PCR using the LifeEco ver. 2.0 (Hangzhou Bioer Technologies).

For one-step real-time RT-ORNi-PCR, a double-labeled fluorescent probe (0.2 µM) was added to the reaction mixture before amplification using the CFX Connect Real-Time PCR Detection System (Bio-Rad Laboratories).

### Investigation of Blocking Mechanism by ORN Using One-Step RT-ORNi-PCR

To examine the blocking effect of ORN on RT, a reaction mixture (10 µL) containing ORN, 25 ng of RNA, and reagents from the iTaq Universal Probes One-Step Kit (Bio-Rad Laboratories) was prepared according to the manufacturer's protocol. RT was performed at 50°C, and the obtained cDNA was purified using FastGene Gel/PCR Extraction Kit (Nippon Genetics, Tokyo, Japan). After that, a reaction mixture (10 µL) containing the purified cDNA and THUNDERBIRD Probe qPCR Mix (TOYOBO) was prepared according to the manufacturer's protocol. PCR was performed using the Mastercycler nexus (Eppendorf). To evaluate the blocking effect of ORN during the PCR step, a reaction mixture (10 µL) containing 25 ng of RNA and reagents from the iTaq Universal Probes One-Step Kit (Bio-Rad Laboratories) was prepared according to the manufacturer's protocol. After the RT reaction at 50°C, ORN was added, and the PCR step was performed.

### Example 1

### ORNi-PCR with Taq DNA Polymerase

### Experimental Example 1-1

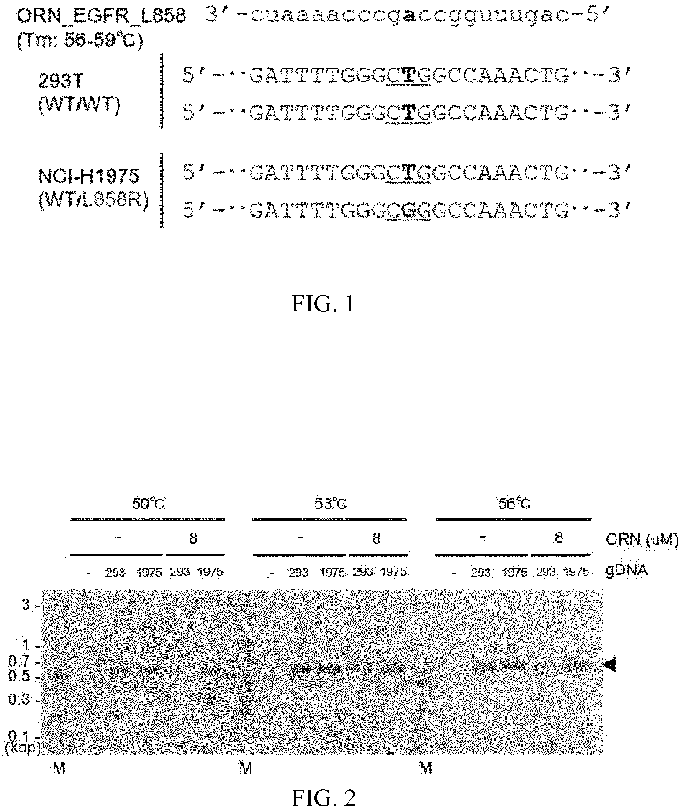

The applicability of three types of Taq DNA polymerase-the THUNDERBIRD Probe qPCR Mix (TOYOBO), the GoTaq Probe qPCR Master Mix (Promega), and The Premix Taq (TaKaRa Taq Version) (Takara Bio)-in ORNi-PCR was tested using gDNA extracted from each of 293T and NCI-H1975 cells along with ORN. 293T cells have WT EGFR, whereas NCI-H1975 cells carry an L858R mutation in one allele of the EGFR gene (hereinafter referred to as "L858R EGFR") (Fig. 1). The ORN sequence (ORN_EGFR_L858, SEQ ID NO: 1) is designed to target WT EGFR sequence corresponding to L858 (Fig. 1).

ORNi-PCR was performed using Taq DNA polymerase with 5' to 3' exonuclease activity. The pair of primers used was hEGFR-Exon21-F (SEQ ID NO: 2) and hEGFR-Exon21-R (SEQ ID NO: 3). Table 1 shows the PCR conditions in the THUNDERBIRD Probe qPCR Mix and the GoTaq Probe qPCR Master Mix, and Table 2 shows the PCR conditions in The Premix Taq.

**[Table 1]**

| Temperature | Time | Cycle |
|---|---|---|
| 95°C | 1 min | 1 |
| 95°C | 15 sec | 30 |
| 50, 53 or 56°C | 1 min | |

**[Table 2]**

| Temperature | Time | Cycle |
|---|---|---|
| 98°C | 10 sec | 30 |
| 50, 53 or 56°C | 1 min | |

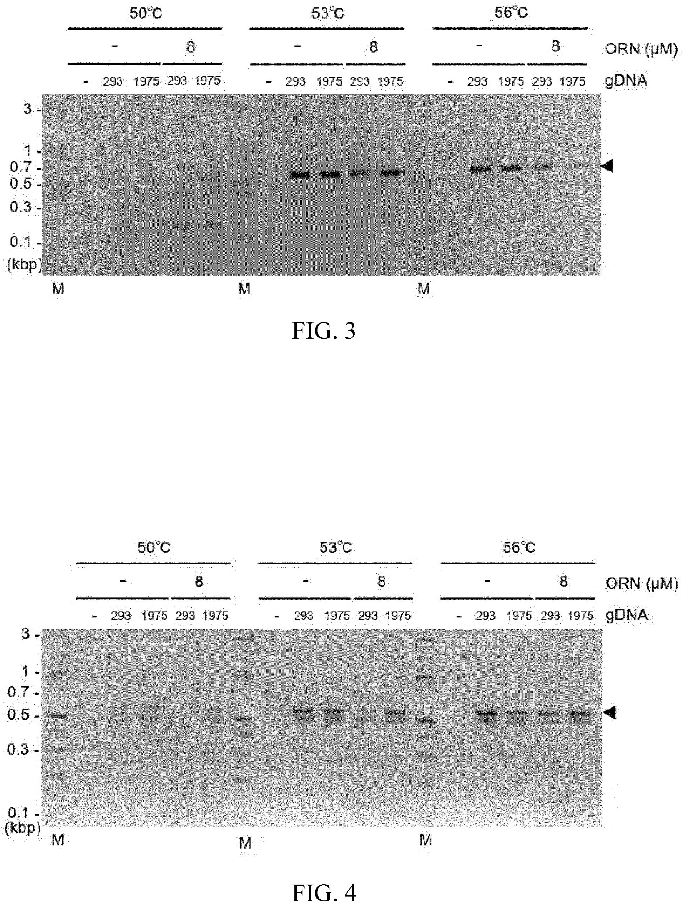

Figs. 2 to 4 show electrophoresis images of nucleic acids amplified using the THUNDERBIRD Probe qPCR Mix, the GoTaq Probe qPCR Master Mix and the Premix Taq, respectively.

In our previous study, we demonstrated that under optimized experimental conditions (i.e., 0.5 to 2 µM ORN; during the annealing and elongation steps at 56 to 59°C), the use of ORN_EGFR_L858, which targets the WT EGFR sequence corresponding to L858, together with KOD DNA polymerase without 5' to 3' exonuclease activity, effectively suppressed amplification of the WT EGFR sequence, while amplification of the L858R sequence was not suppressed (not shown).

As shown in Figs. 2 to 4, in ORNi-PCR using Taq DNA polymerase with 5' to 3' exonuclease activity, no such inhibitory effect was observed even when the ORN concentration was increased and the annealing and elongation steps were performed at 56°C (Figs. 2 to 4). This suggests that the hybridized ORN was removed during DNA elongation.

In contrast, when the annealing and elongation steps were performed at 50°C, this ORN specifically suppressed amplification of the WT EGFR gene (Figs. 2-4).

### Experimental Example 1-2

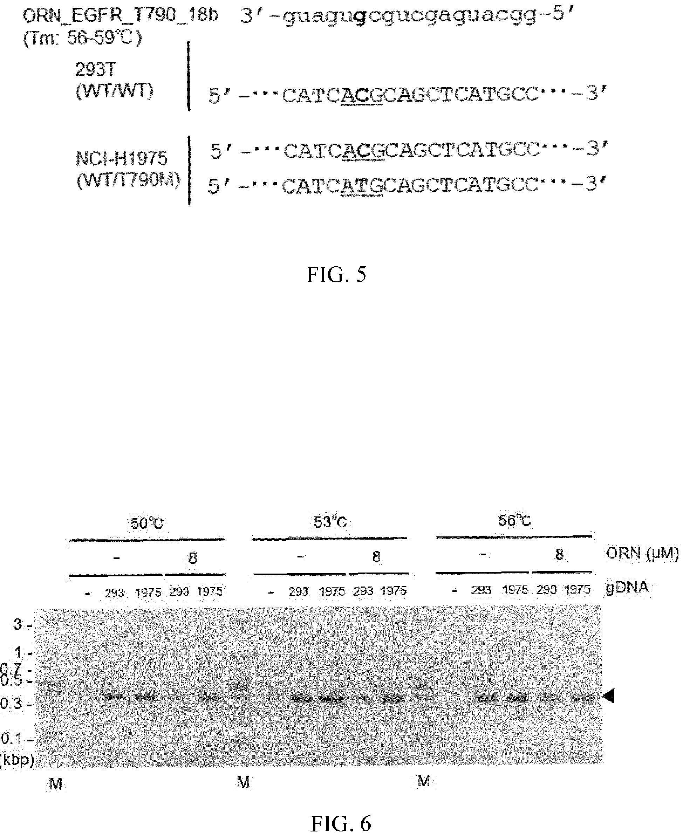

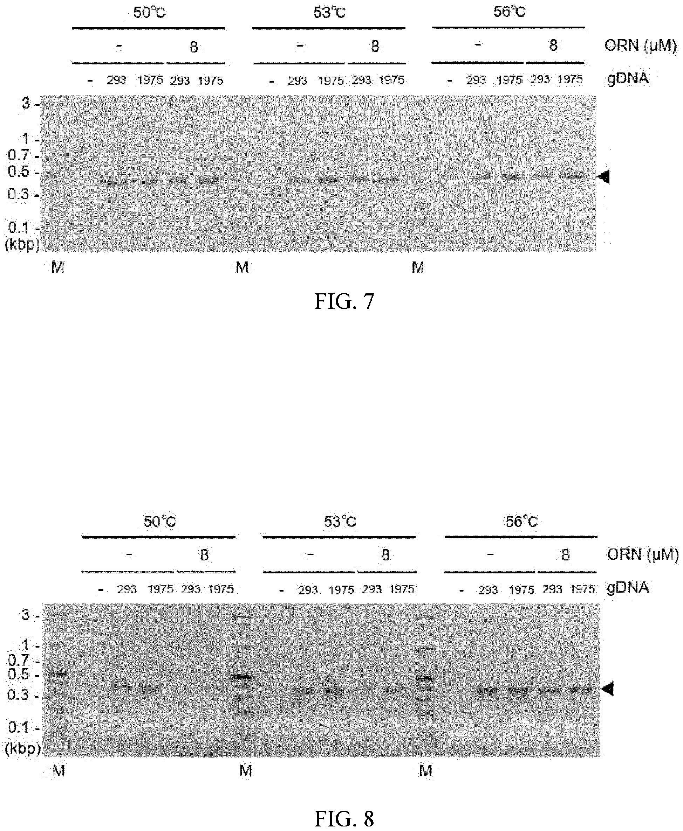

ORNi-PCR was performed using ORN (ORN_EGFR_T790_18b, (SEQ ID NO: 4)) targeting another position (corresponding to T790) within the WT EGFR gene (Fig. 5). ORNi-PCR was performed in the same manner as in Experimental Example 1-1, except that NCI-H1975 cells carrying a T790M mutation in one allele of the EGFR gene (hereinafter referred to as "T790M EGFR"), ORN_EGFR_T790_18b, and the hEGFR-Exon20 primer pair (hEGFR-Exon20-F3 (SEQ ID NO: 32) and hEGFR-Exon20-R3 (SEQ ID NO: 33)) were used. In all Taq DNA polymerases, DNA amplification was specifically inhibited at 50°C (the annealing and elongation steps) in a sequence-specific manner (Figs. 6 to 8).

### Experimental Example 1-3

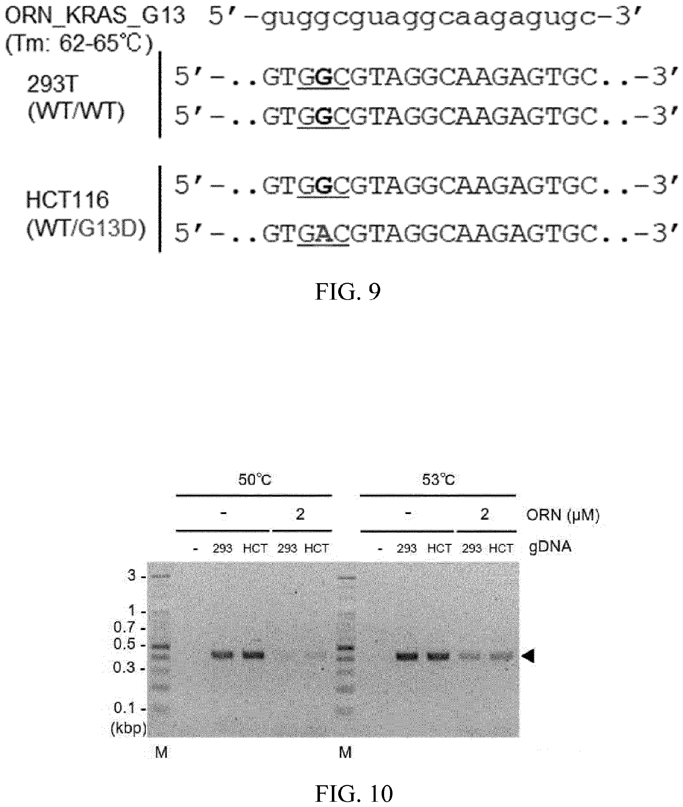

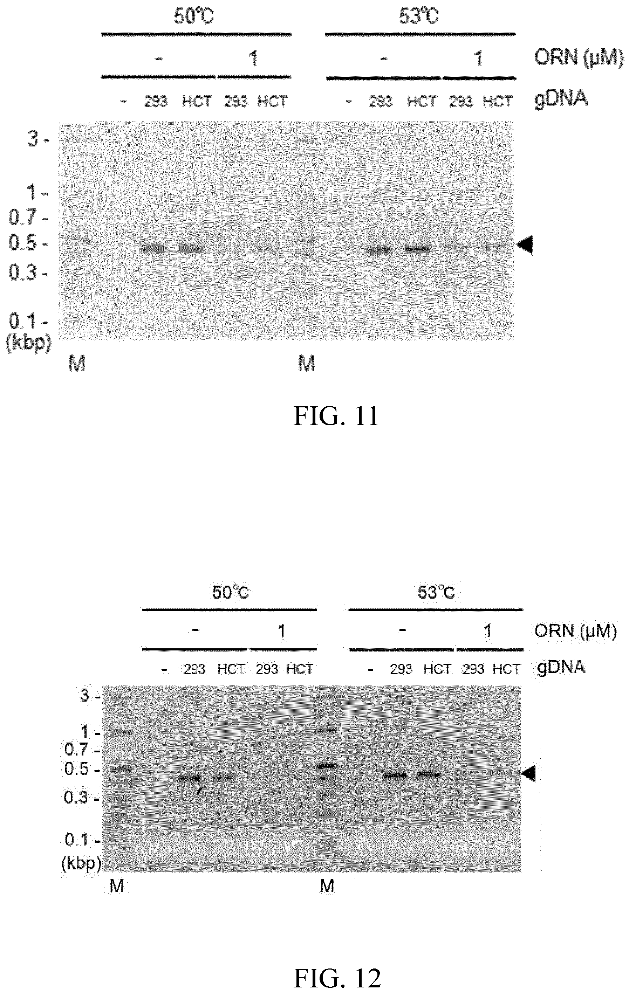

ORNi-PCR was performed using ORN (ORN_KRAS_G13, (SEQ ID NO: 5)) targeting the wild-type KRAS (WT KRAS) gene (corresponding to G13) (Fig. 9). ORNi-PCR was performed in the same manner as in Experimental Example 1-1, except that HCT116 cells carrying a G13D mutation in one allele of the KRAS gene (hereinafter referred to as "G13D KRAS"), ORN_KRAS_G13, and the hKRAS primer pair (hKRAS-F4 (SEQ ID NO: 6) and hKRAS-R2 (SEQ ID NO: 7)) were used. In all Taq DNApolymerases, DNA amplification was specifically inhibited at 50°C (the annealing and elongation steps) in a sequence-specific manner (Figs. 10 to 12).

### Conclusion of Example 1

These results indicate that Taq DNApolymerases with 5' to 3' exonuclease activity are compatible with ORNi-PCR performed at a relatively low temperature (around 50°C) during the annealing + extension step.

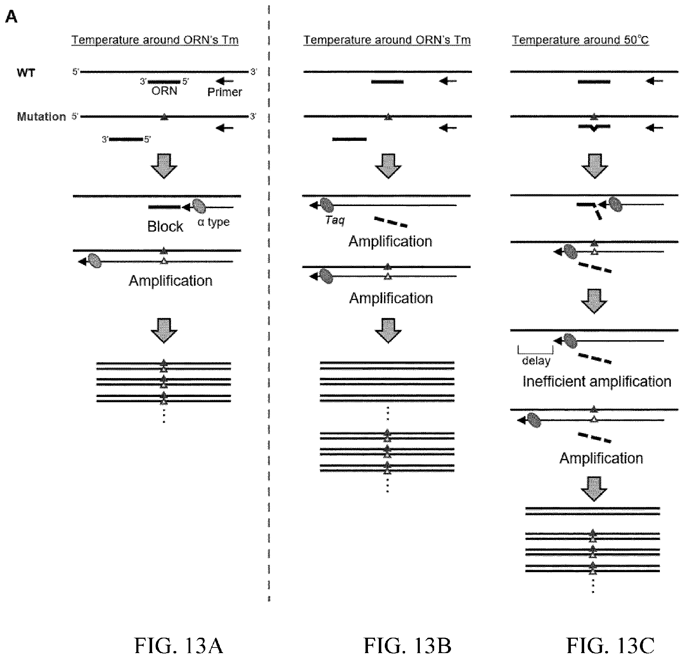

Unlike DNA polymerases without 5' to 3' exonuclease activity (α type), the Taq DNA polymerase with 5' to 3' exonuclease activity actively removes hybridized ORNs when DNA extension is carried out near the melting temperature (Tm) of ORN/DNA hybridization (e.g., 56°C for ORN_EGFR_L858) (Figs. 13A and 13B).

Removal of hybridized ORNs could be inhibited at temperatures lower than Tm (around 50°C). At such temperatures, Taq DNA polymerase cannot actively remove hybridized ORNs during DNA elongation (Fig. 13C). In this case, ORNs that mismatched with the target sequence were removed more effectively than ORNs that hybridized completely, resulting in differences in DNA amplification efficiency (Fig. 13C).

### Example 2

### Sequence-Specific Suppression of DNA Amplification by ORNi-PCR Using GeneAce qPCR Master Mix II

### Experimental Example 2-1

GeneAce qPCR Master Mix II (hereinafter referred to as "GeneAce Taq"), which contains a Taq DNA polymerase different from that used in Example 1, was evaluated. ORNi-PCR was performed in the same manner as in Experimental Example 1-1, except for the use of GeneAce Taq and the PCR conditions. Table 3 shows the PCR conditions.

**[Table 3]**

| Temperature | Time | Cycle |
|---|---|---|
| 95°C | 10 min | 1 |
| 95°C | 30 sec | 30 |
| 59 or 62°C | 1 min | |

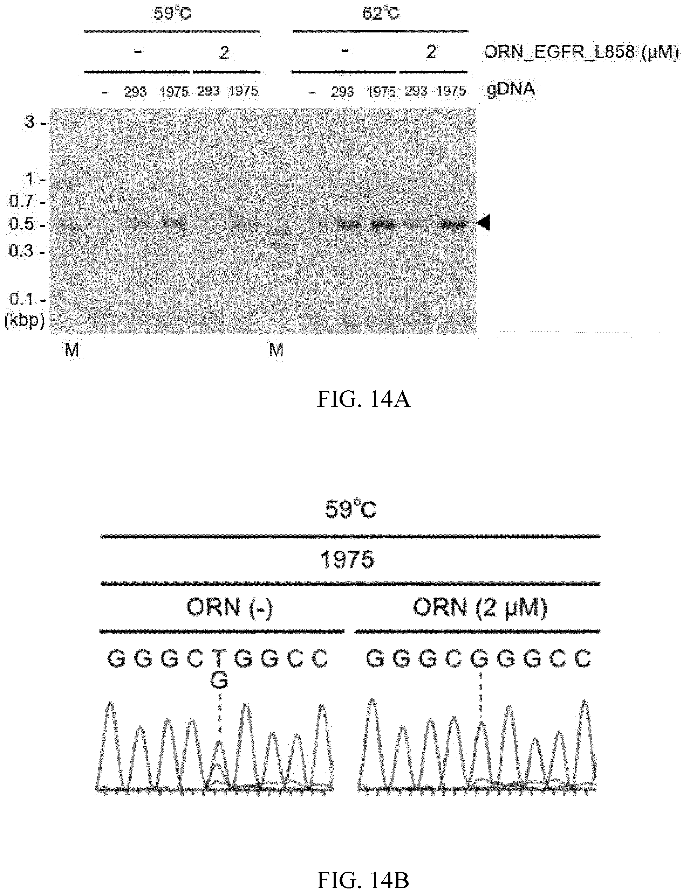

Fig. 14A shows an electrophoresis image of nucleic acids amplified using GeneAce Taq. Fig. 14B shows the results of DNA sequence analysis of the amplification products indicated by an arrow in Fig. 14A. As shown in Figs. 14A and 14B, amplification of the WT EGFR gene was specifically suppressed in a sequence-specific manner under specific experimental conditions (i.e., 2 µM of ORN_EGFR_L858; during the annealing and elongation steps at 59°C).

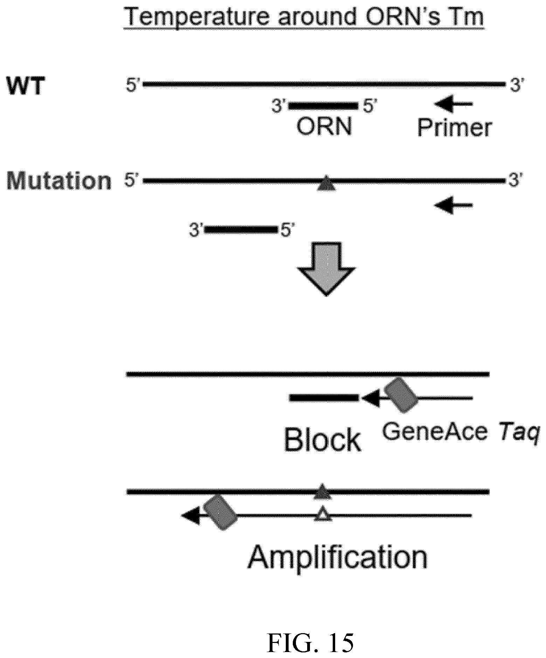

Since these experimental conditions are the same as the optimal conditions for ORNi-PCR with KOD DNA polymerase, the mode of inhibition is considered identical (Figs. 13A and 15).

### Experimental Example 2-2

ORNi-PCR was performed using ORN (ORN_EGFR_T790_18b, (SEQ ID NO: 4)) targeting another position (corresponding to T790) within the WT EGFR gene. ORNi-PCR was performed in the same manner as in Experimental Example 1-2, except for the use of GeneAce Taq and the PCR conditions. Table 4 shows the PCR conditions.

**[Table 4]**

| Temperature | Time | Cycle |
|---|---|---|
| 95°C | 10 min | 1 |
| 95°C | 15 sec | 40 |
| 59 or 62°C | 1 min | |

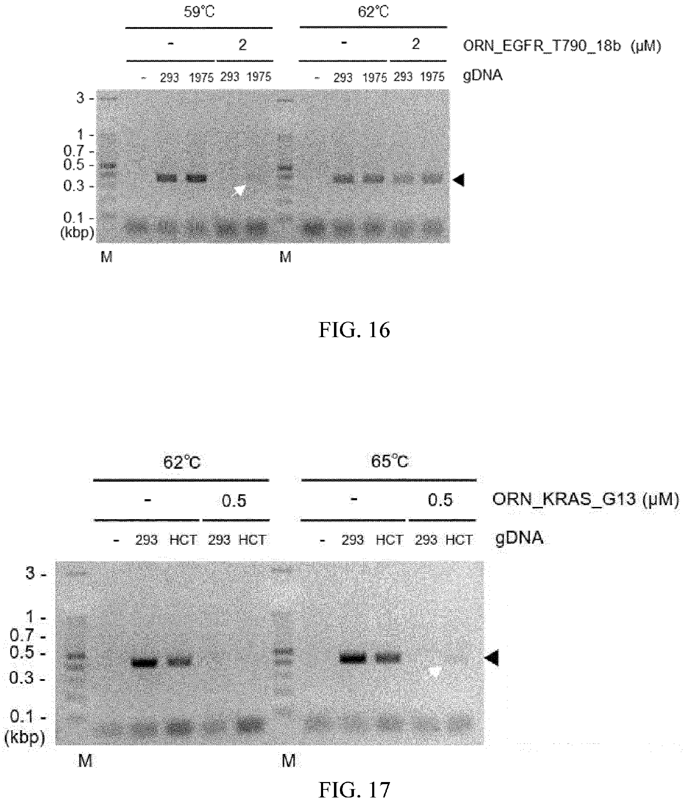

DNA amplification at 59°C (the annealing and elongation steps) was also suppressed in a sequence-specific manner when using GeneAce Taq (Fig. 16).

### Experimental Example 2-3

ORNi-PCR was performed using ORN (ORN_KRAS_G13 (SEQ ID NO: 5)) targeting the WT KRAS gene (corresponding to G13). ORNi-PCR was performed in the same manner as in Experimental Example 1-3, except for the use of GeneAce Taq and the PCR conditions. Table 5 shows the PCR conditions.

**[Table 5]**

| Temperature | Time | Cycle |
|---|---|---|
| 95°C | 10 min | 1 |
| 95°C | 15 sec | 40 |
| 62 or 65°C | 1 min | |

DNA amplification at 65°C (the annealing and elongation steps) was also suppressed in a sequence-specific manner when using GeneAce Taq (Fig. 17).

### Experimental Example 2-4

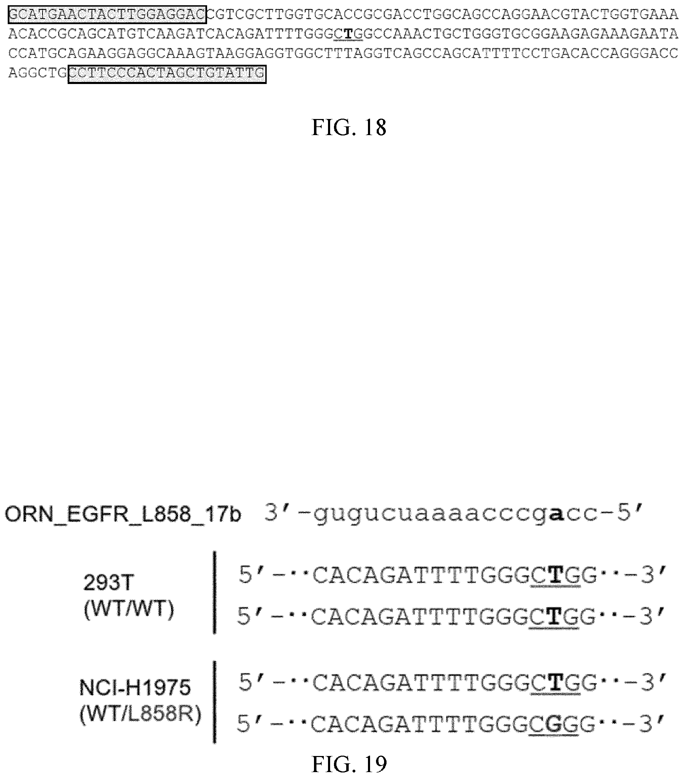

GeneAce Taq is a reagent optimized for real-time PCR using double-labeled fluorescent probes. To investigate its application for real-time ORNi-PCR, primer positions were optimized to amplify approximately 0.2 kbp of the EGFR gene. Fig. 18 shows the nucleotide sequence of the nucleic acid amplified by the optimized primer pair (SEQ ID NO: 8). The box upstream (5' side) of the sequence indicates the position of the forward primer (hEGFR-Exon21-F8, SEQ ID NO: 9), the box downstream (3' side) indicates the position of the reverse primer (hEGFR-Exon21-R8, SEQ ID NO: 10), and the underlined region indicates the position corresponding to L858.

The concentrations of ORN_EGFR_L858 were 0 µM, 3 µM and 6 µM to specifically suppress the WT EGFR sequence. Another ORN targeting the WT EGFR sequence corresponding to L858 (ORN_EGFR_L858_17b (SEQ ID NO: 11), Fig. 19) was also tested. Table 6 shows the PCR conditions.

**[Table 6]**

| Temperature | Time | Cycle |
|---|---|---|
| 95°C | 10 min | 1 |
| 95°C | 20 sec | 40 |
| 56 or 59°C | 50 sec | |

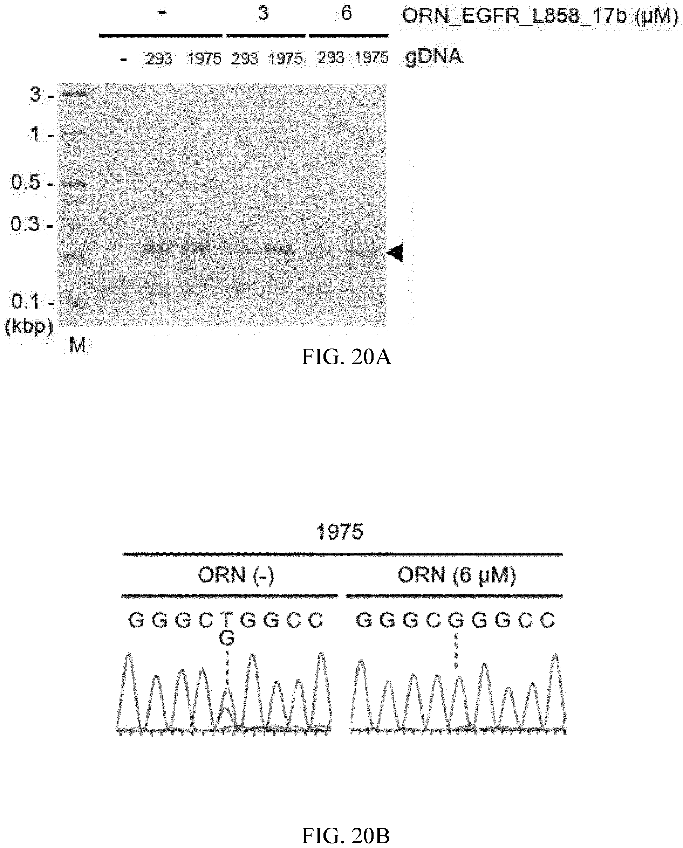

As shown in Figs. 20A and 20B, this ORN at also specifically suppressed amplification of the WT EGFR sequence at 6 µM during the annealing and elongation steps at 56°C.

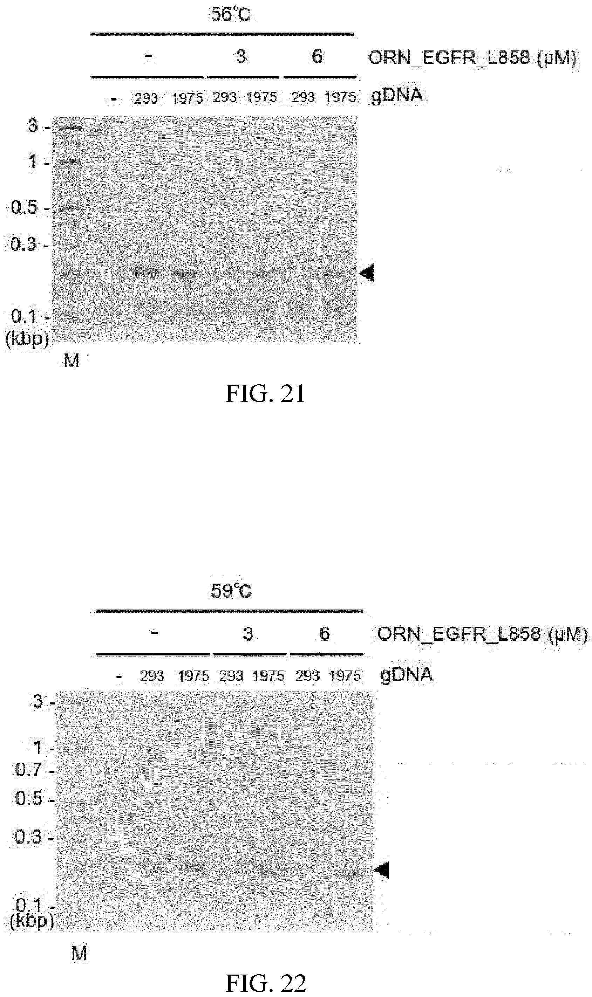

Figs. 21 and 22 show electrophoresis images related to ORN_EGFR_L858 (during the annealing and elongation steps at 56°C) and ORN_EGFR_L858 (during the annealing and elongation steps at 59°C), respectively. These ORNs also specifically suppressed amplification of the WT EGFR sequence at 6 µM during the annealing and elongation steps at 56°C.

Thus, it is clear that GeneAce Taq is applicable to real-time ORNi-PCR with double-labeled fluorescent probes.

### Example 3

### Real-Time ORNi-PCR Using GeneAce Taq in Combination with Double-Labeled Fluorescent Probes

### Experimental Example 3-1

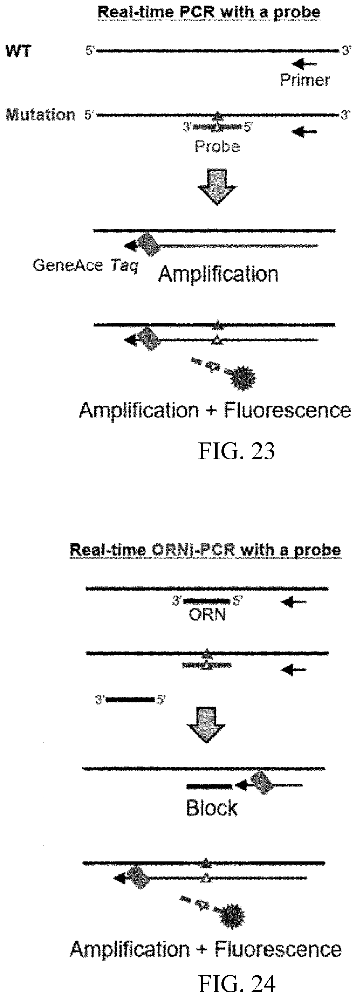

Real-time ORNi-PCR with double-labeled fluorescent probes was performed using GeneAce Taq. Fig. 23 shows a schematic diagram of conventional real-time PCR using double-labeled fluorescent probes, and Fig. 24 shows a schematic diagram of real-time ORNi-PCR using double-labeled fluorescent probes. The primers used were hEGFR-Exon21-F8 and hEGFR-Exon21-R8.

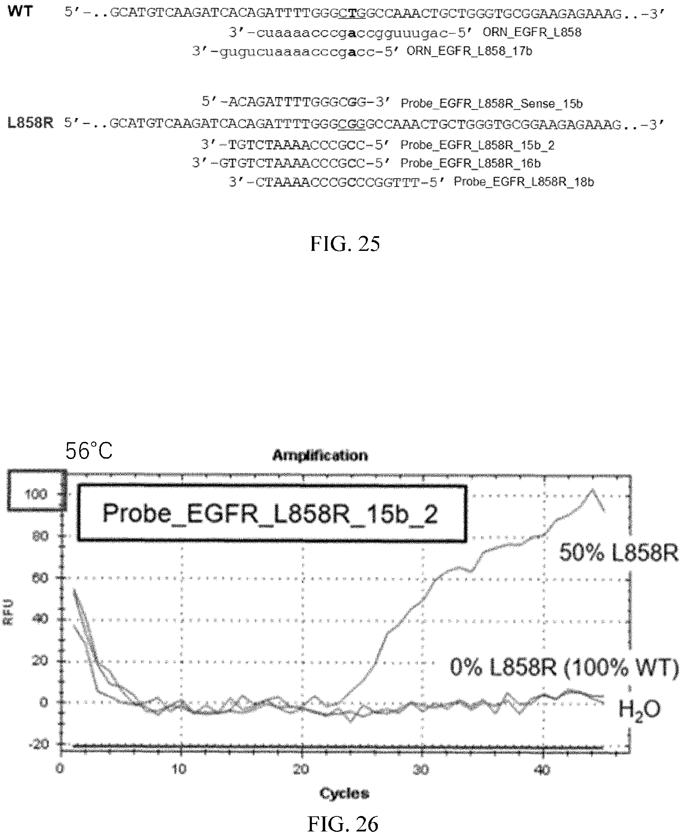

Various double-labeled fluorescent probes targeting the L858R EGFR mutation were designed (Fig. 25 and Table 7).

**[Table 7]**

| Probe name | Nucleotide sequence (5' → 3') | SEQ ID Nos |
|---|---|---|
| Probe_EGFR_L858R_Sense_15b | ACAGATTTTGGGCGG | SEQ ID NO: 12 |
| Probe_EGFR_L858R_15b_2 | CCGCCCAAAATCTGT | SEQ ID NO: 13 |
| Probe_EGFR_L858R_16b | CCGCCCAAAATCTGTG | SEQ ID NO: 14 |
| Probe_EGFR_L858R_18b | TTTGGCCCGCCCAAAATC | SEQ ID NO: 15 |

Table 8 shows the PCR conditions.

**[Table 8]**

| Temperature | Time | Cycle |
|---|---|---|
| 95°C | 10 min | 1 |
| 95°C | 20 sec | 45 |
| 56 or 59°C | 50 sec | |

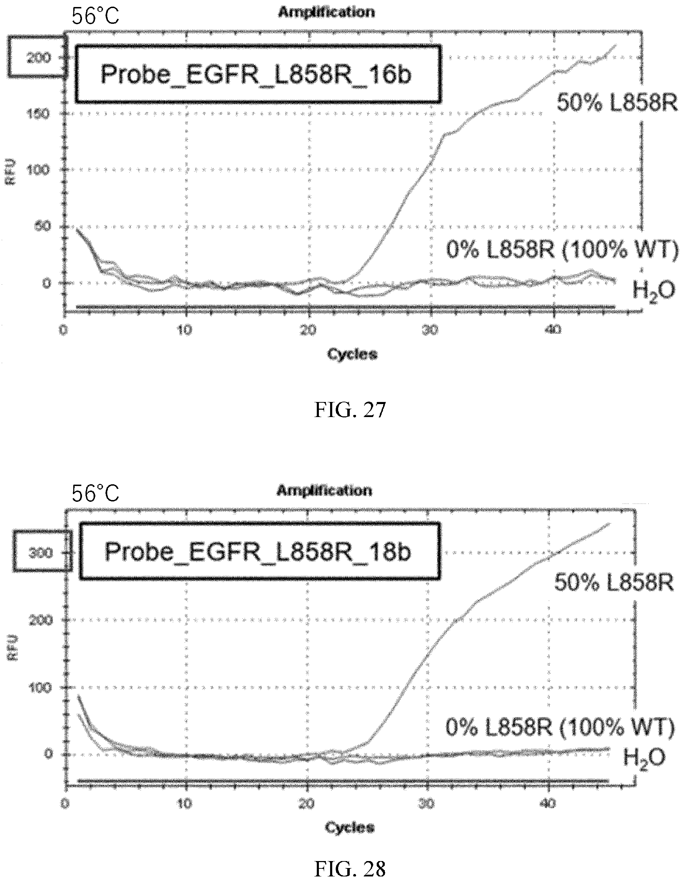

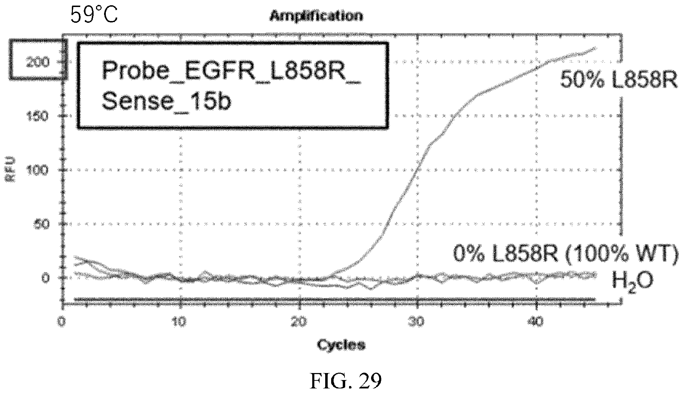

Results for conventional real-time PCR are shown in Figs. 26 to 29. As shown in Figs. 28 and 29, Probe_EGFR_L858R_18b and Probe_EGFR_L858R_Sense_15b detected target mutations more specifically and effectively in conventional real-time PCR using GeneAce Taq.

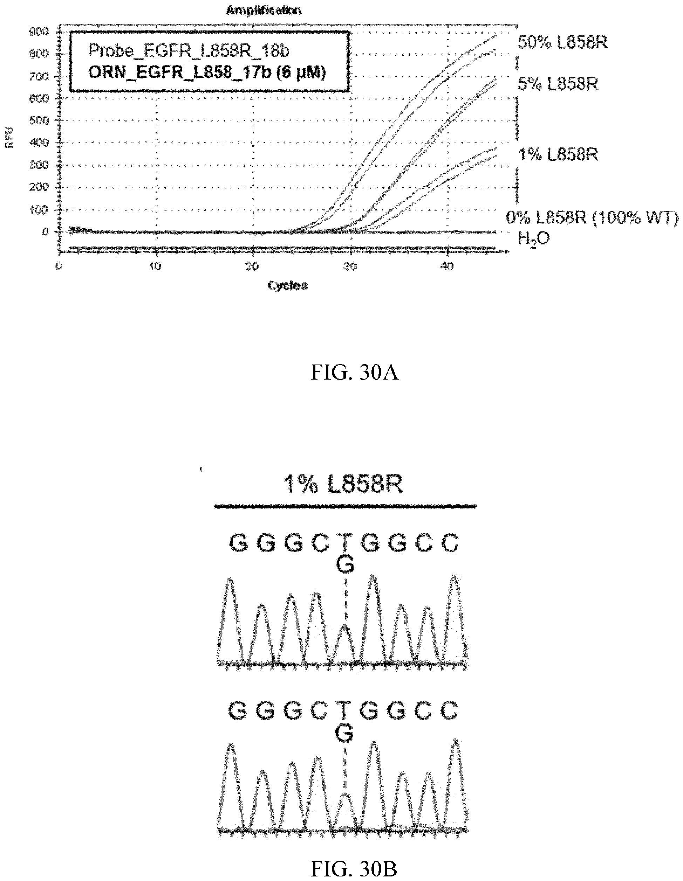

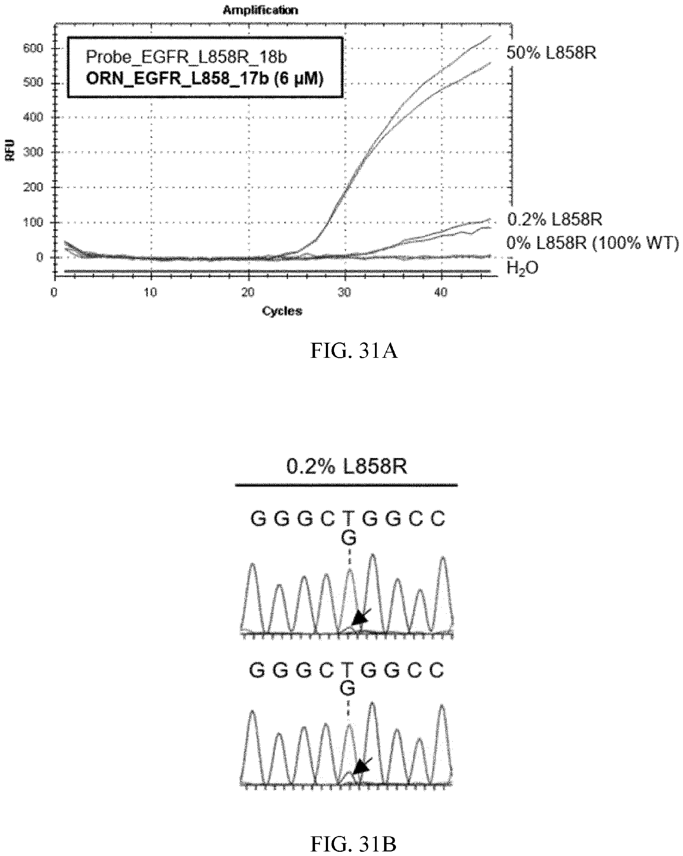

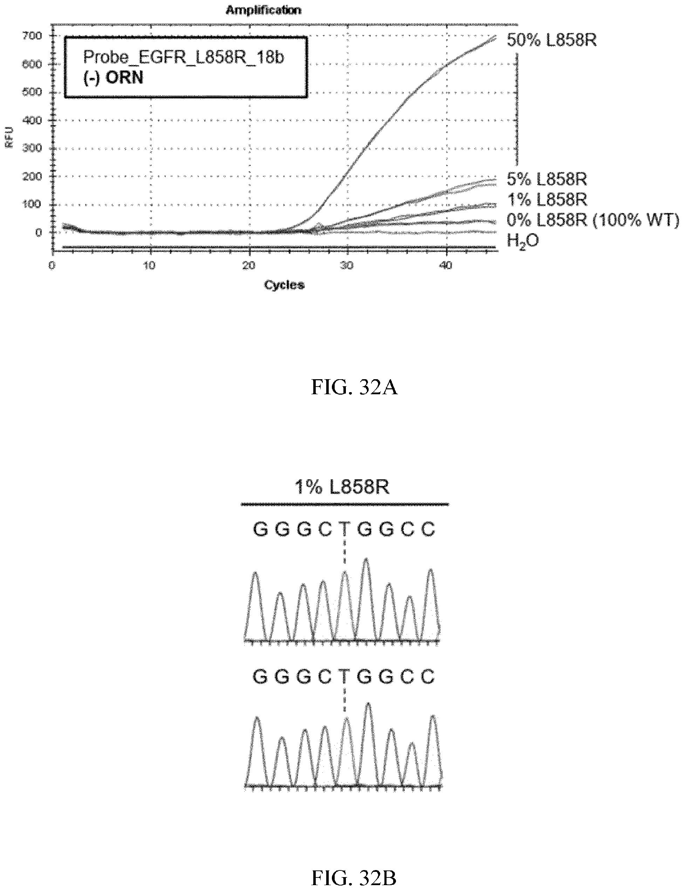

Results for real-time ORNi-PCR and real-time PCR are shown in Figs. 30A and 30B to 32A and 32B. Real-time ORNi-PCR using ORN_EGFR_L858_17b and Probe_EGFR_L858R_18b led to strong fluorescence detection even when the L858R EGFR gene was present in only 0.2 to 1% of the total EGFR template (6 to 33 copies of L858R EGFR) (Figs. 30A, 30B, 31A and 31B).

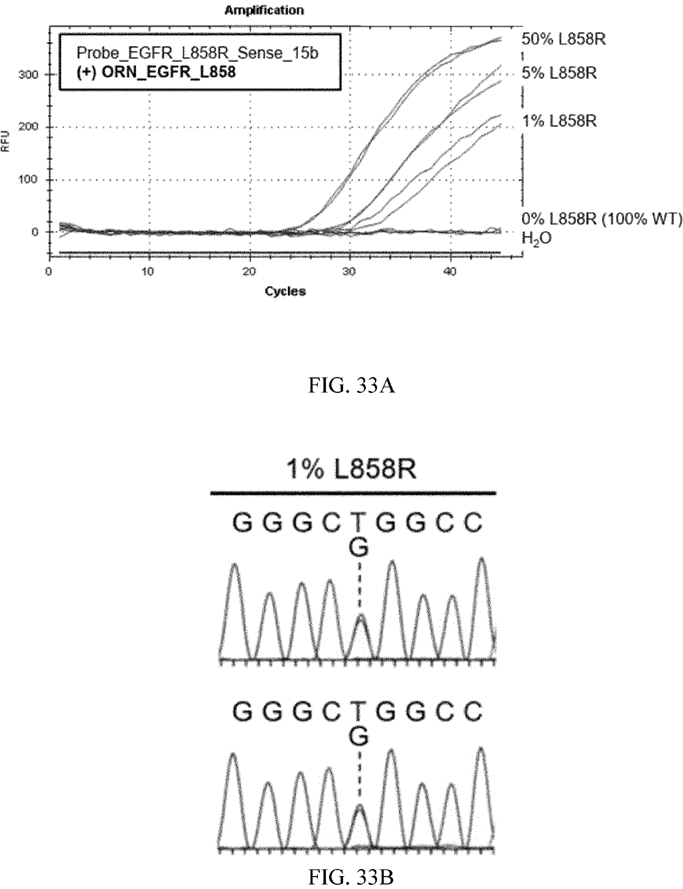

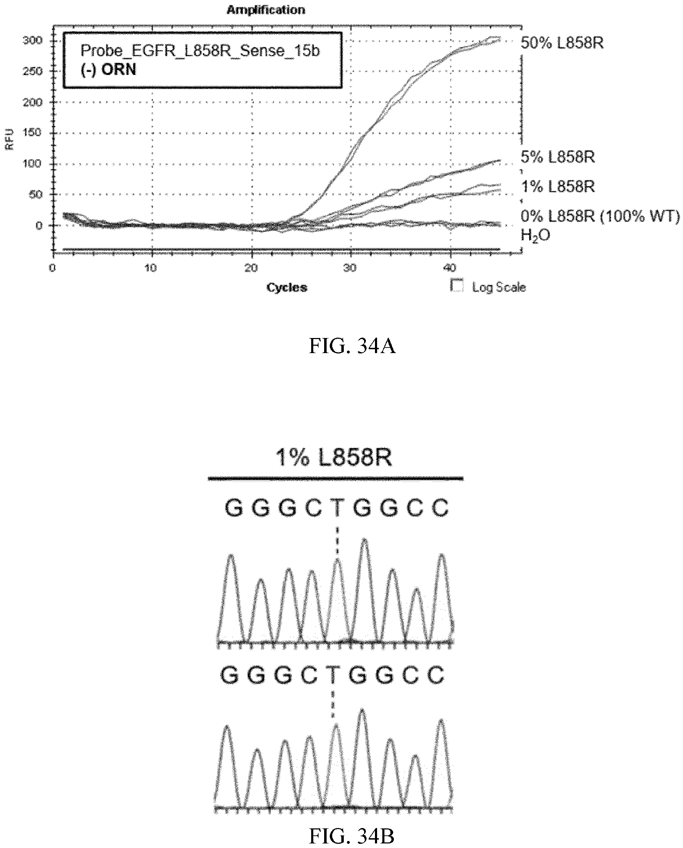

DNA sequence analysis confirmed amplification of the mutant sequence from the real-time ORNi-PCR amplicon. However, the WT EGFR sequence was also amplified with incomplete suppression (Figs. 30B and 31B). Similar results were obtained in real-time ORNi-PCR using ORN_EGFR_L858 and Probe_EGFR_L858R_Sense_15b (Figs. 33A and B). On the other hand, the conventional real-time PCR using Probe_EGFR_L858R_18b or Probe_EGFR_L858R_Sense_15b showed limited amplification of fluorescence intensity when the mutation accounted for 1% of the total EGFR template (Figs. 32A and 34A). DNA sequence analysis did not confirm the presence of this mutation (Figs. 32B and 34B).

Thus, the real-time ORNi-PCR can preferentially amplify specific sequences (e.g., mutant sequences), which can then be detected with a double-labeled fluorescent probe, whereas the conventional real-time PCR results may be false positives.

### Experimental Example 3-2

DNA extracted from formalin-fixed paraffin-embedded (FFPE) specimens is used in the medical field for cancer diagnosis. Therefore, DNA extracted from commercially available FFPE human specimens that mimic patient specimens was used for real-time ORNi-PCR.

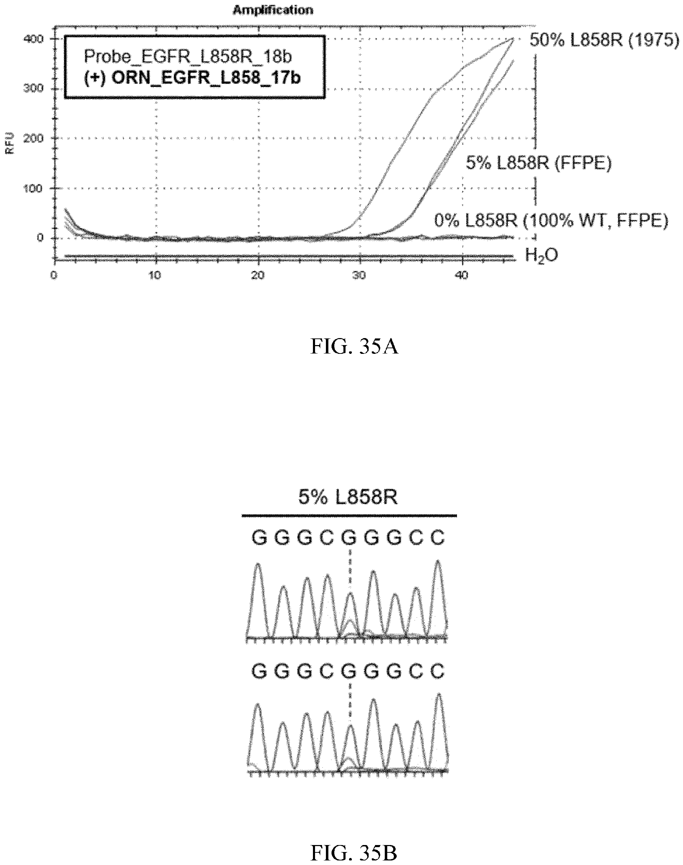

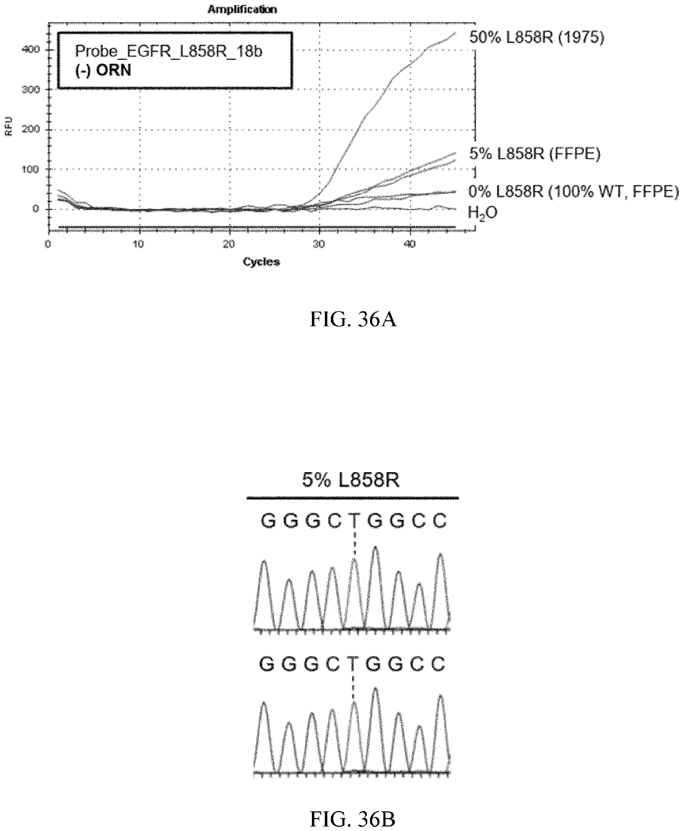

As shown in Figs. 35A, 35B, 36A and 36B, the real-time ORNi-PCR with Probe_EGFR_L858R_18b and ORN_EGFR_L858_17b was able to more specifically and stably amplify L858R EGFR in extracted DNA than the conventional real-time PCR.

### Conclusion of Example 3

In summary, we have successfully performed the real-time ORNi-PCR with the double-labeled fluorescent probes using GeneAce Taq and confirmed that it is advantageous over the conventional real-time PCR with respect to sensitivity and specificity in detecting single nucleotide differences. If necessary, a definitive diagnosis can be made by DNA sequencing analysis based on amplicons generated by real-time ORNi-PCR.

### Example 4

### Mechanism of DNA Elongation Suppression Mediated by ORN Using GeneAce Taq Experimental Example 4-1

To examine the effect of chemically modified Taq DNA polymerase/dUTP on the suppression of target DNA amplification by ORNs, the chemically modified Taq DNA polymerase used in GeneAce Taq (Hot-Start Gene Taq NT) and the unmodified version thereof (Gene Taq NT) were purchased from the same company.

ORNi-PCR was performed in the same manner as in Experimental Example 1-1, except for the use of chemically modified Taq DNA polymerase (Hot-Start Gene Taq NT), primer pairs (hEGFR-Exon21-F8 (SEQ ID NO: 9) and hEGFR-Exon21-R8 (SEQ ID NO:10)) and PCR conditions. Table 9 shows the PCR conditions.

**[Table 9]**

| Temperature | Time | Cycle |
|---|---|---|
| 95°C | 5 min | 1 |
| 95°C | 20 sec | 35 |
| 56°C | 50 sec | |

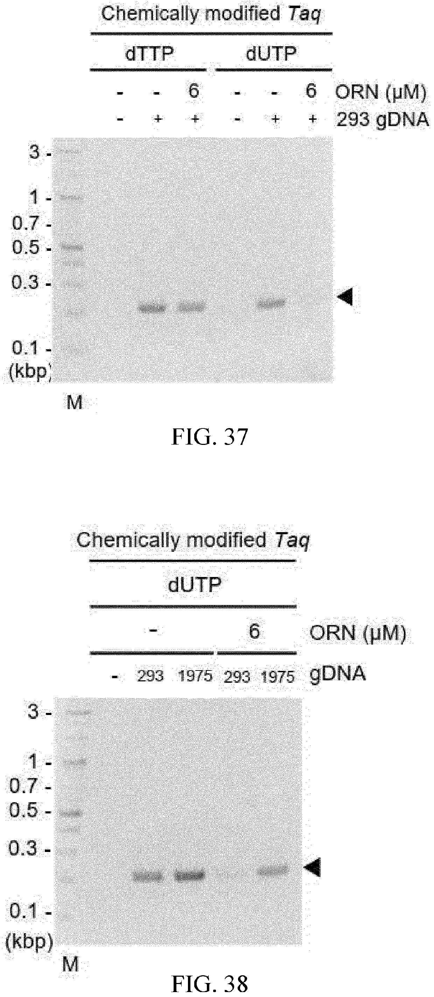

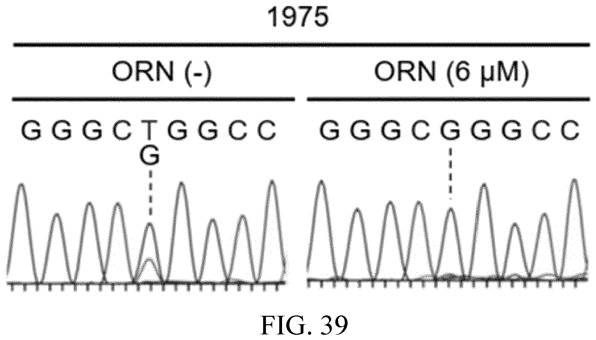

As shown in Figs. 37 to 39, ORN_EGFR_L858_17b specifically suppressed amplification of WT EGFR when the chemically modified Taq DNA polymerase was used in combination with dUTP (without dTTP).

### Experimental Example 4-2

Another chemically modified Taq DNA polymerase (AmpliTaq Gold 360) was also used in the experiment. ORNi-PCR was performed in the same manner as in Experimental Example 4-1, except for the use of AmpliTaq Gold 360 and PCR conditions. Table 10 shows the PCR conditions.

**[Table 10]**

| Temperature | Time | Cycle |
|---|---|---|
| 95°C | 10 min | 1 |
| 95°C | 15 sec | 30 |
| 56°C | 30 sec | |

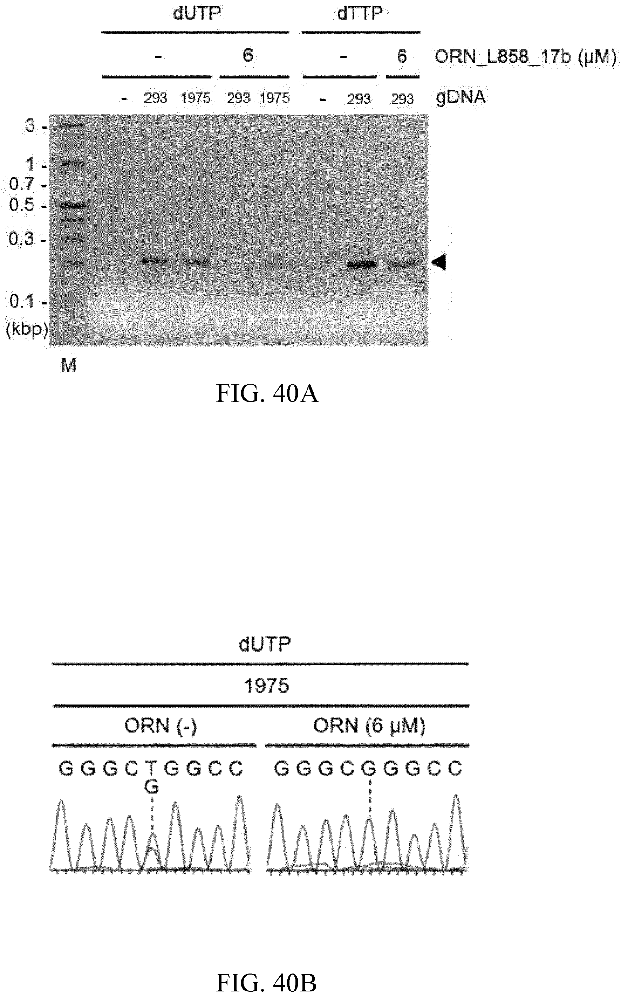

Similar results were obtained for AmpliTaq Gold 360, as shown in Figs. 40A and 40B.

### Comparative Example 4-1

The unmodified Taq DNA polymerase (Gene Taq NT) was used in the experiment. ORNi-PCR was performed in the same manner as in Experimental Example 4-1, except for the use of Gene Taq NT.

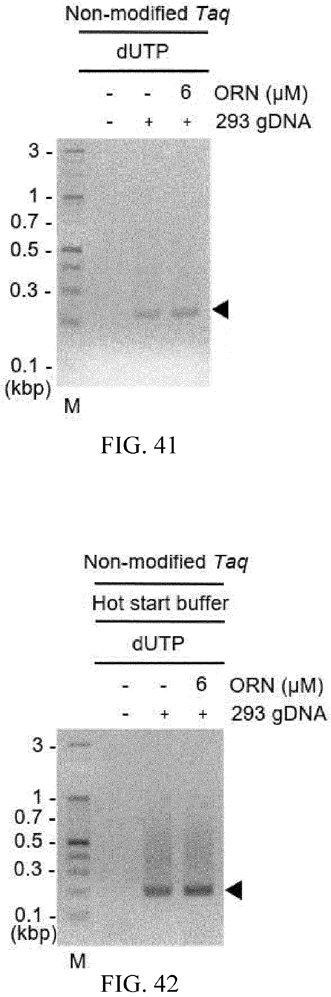

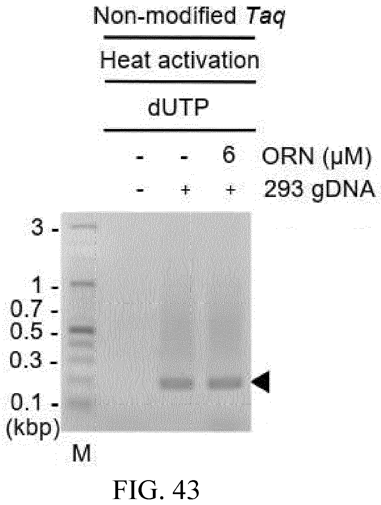

In contrast, DNA amplification by the unmodified Taq DNA polymerase (Gene Taq NT) was not inhibited by the presence of dUTP, the reaction buffer of the Hot-Start Gene Taq NT, or the long heat treatment (5 min) required to activate the chemically modified Taq DNA polymerase (Figs. 41 to 43).

### Results of Example 4

These results clearly demonstrate the importance of using the chemically modified Taq DNA polymerase in combination with dUTP to suppress the target DNA amplification by ORN.

### Example 5

### Inhibition of DNA Elongation Reaction by Taq DNA Polymerase with Phosphorothioate-Modified ORN

### Experimental Example 5-1

Phosphorothioate modification enhances the resistance of RNA to degradation by ribonucleases. This property can be utilized to stabilize antisense oligonucleotides, enabling their use as oligonucleotide therapeutics.

Whether phosphorothioate modification increases the resistance of ORNs to degradation by the 5' to 3' exonuclease activity of Taq DNA polymerase was investigated.

ORN_EGFR_L858 with phosphorothioate modifications at all phosphodiester bonds (ORN_EGFR_L858_S_All) and ORN_EGFR_L858 with partial modification at the five phosphodiester bonds at the 5' end (ORN_EGFR_L858_S_5) were synthesized (Table 11).

**[Table 11]**

| ORN Name | Nucleotide sequence (5' → 3') | SEQ ID Nos |
|---|---|---|
| ORN_EGFR_L858_S All | c*a*g*u*u*u*g*g*c*c*a*g*c*c*c*a*a*a*a*u*c | SEQ ID No: 16 |
| ORN_EGFR_L858_S_5 | c*a*g*u*u*uggccagcccaaaauc | SEQ ID No: 17 |
| ORN_EGFR_L858_Sense_S_All | g*a*u*u*u*u*g*g*g*c*u*g*g*c*c*a*a*a*c*u*g | SEQ ID No: 18 |

| | | |
|---|---|---|
| *: phosphorothioation | | |

ORNi-PCR was performed in the same manner as in Experimental Example 1-1, except for the PCR conditions. Table 12 shows the PCR conditions.

**[Table 12]**

| Temperature | Time | Cycle |
|---|---|---|
| 95°C | 1 min | 1 |
| 95°C | 15 sec | 30 |
| 56 or 59°C | 1 min | |

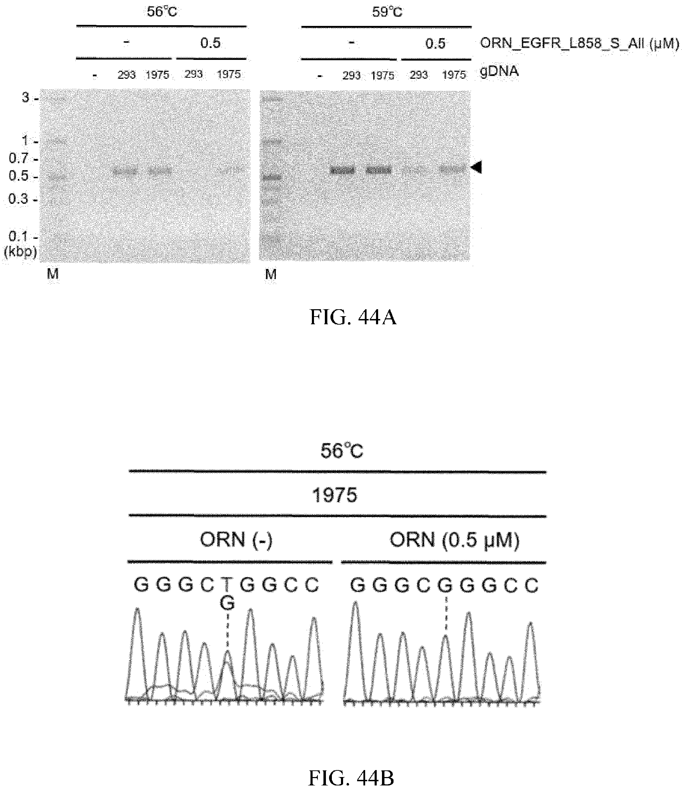

ORN_EGFR_L858_S_All (0.5 µM) suppressed DNA elongation by conventional Taq DNA polymerase (THUNDERBIRD Probe qPCR Mix) in a sequence-specific manner during the annealing and elongation steps at 56°C (Figs. 44A and 44B).

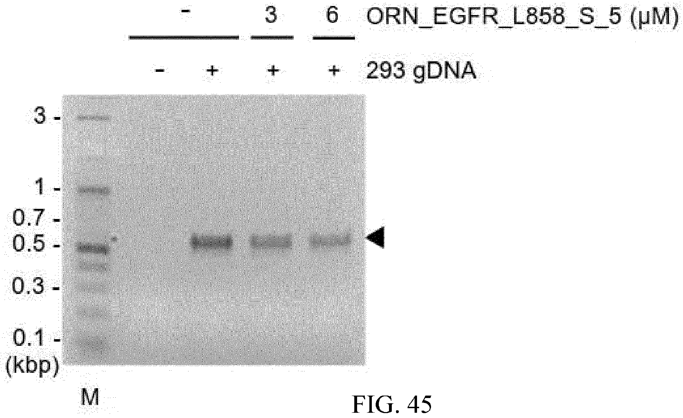

In contrast, ORN_EGFR_L858_S_5 did not exhibit such an inhibitory effect even at high concentrations (Fig. 45).

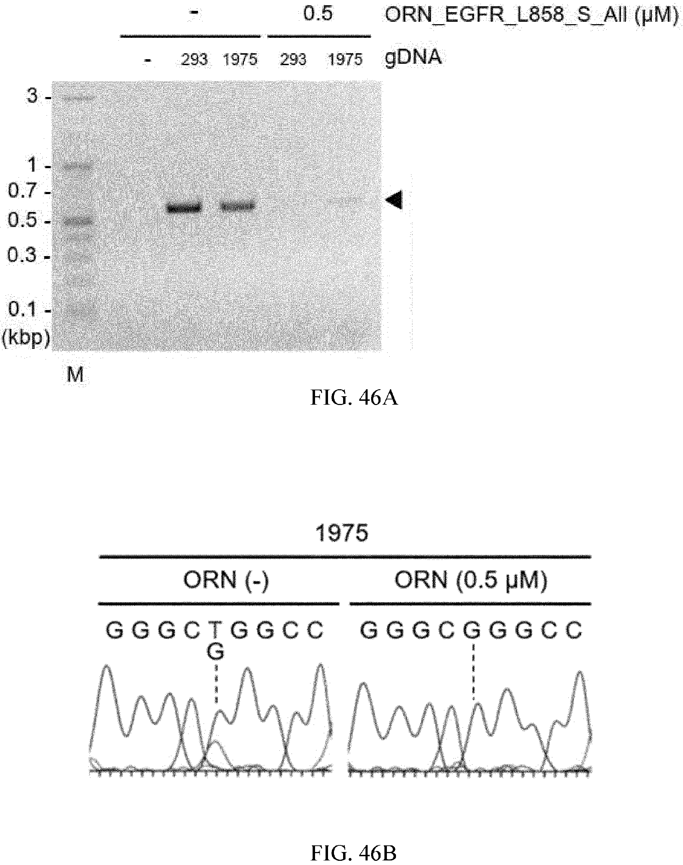

ORN_EGFR_L858_S_All also showed sequence-specific suppression of DNA amplification when another conventional Taq DNA polymerase (GoTaq Probe qPCR Master Mix) was used (Figs. 46A and 46B).

Thus, the fully phosphorothioated ORN (ORN_EGFR_L858_S_All) suppresses DNA elongation by the conventional Taq DNA polymerase in a sequence-specific manner, allowing identification of single nucleotide variations.

### Experimental Example 5-2

For application to real-time ORNi-PCR, the experimental conditions for ORN_EGFR_L858_S_All were optimized. ORNi-PCR was performed in the same manner as in Experimental Example 2-4, except for the use of THUNDERBIRD Probe qPCR Mix and PCR conditions. Table 13 shows the PCR conditions.

**[Table 13]**

| Temperature | Time | Cycle |
|---|---|---|
| 95°C | 1 min | 1 |
| 95°C | 15 sec | 30 |
| 57.5°C | 30 sec | |

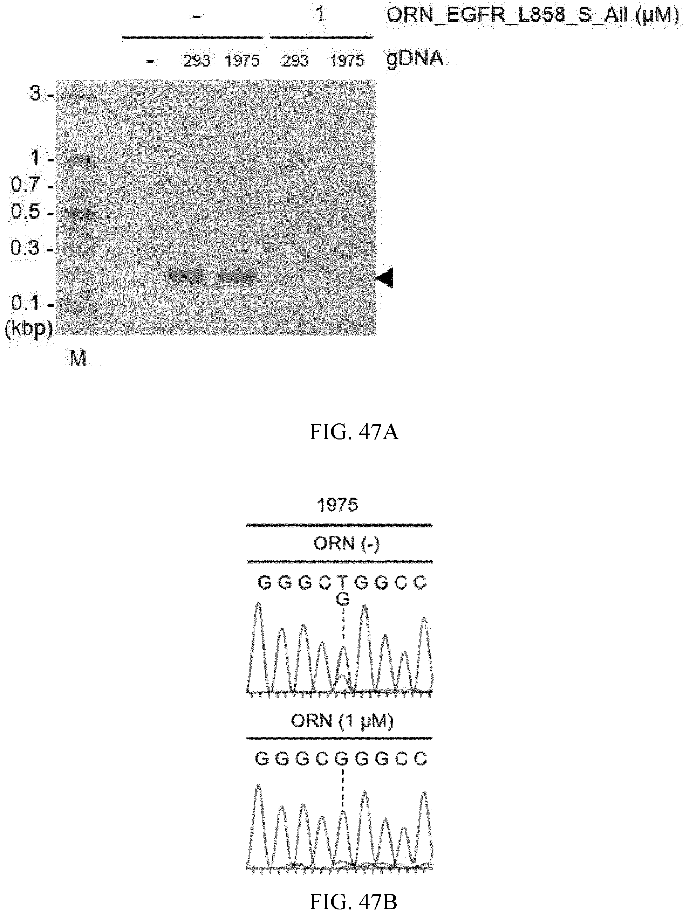

As shown in Figs. 47A and 47B, ORN_EGFR_L858_S_All showed sequence-specific suppression of DNA amplification, even under these conditions.

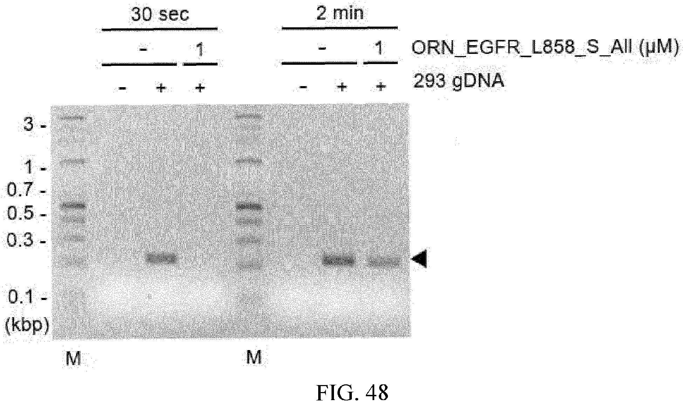

Next, the duration of the annealing and elongation steps was extended from 30 seconds to 2 minutes. Under this condition, the inhibitory efficiency of ORN_EGFR_L858_S_All decreased (Fig. 48).

This suggests that when longer elongation step is used, the fully phosphorothioated ORNs can be removed from the hybridized target DNA by Taq DNA polymerase. Therefore, control of DNA elongation time is important.

### Experimental Example 5-3

Real-time ORNi-PCR using a combination of ORN_EGFR_L858_S_All and Probe_EGFR_L858R_Sense_15b with the conventional Taq DNA polymerase (THUNDERBIRD Probe qPCR Mix), and conventional real-time PCR using Probe_EGFR_L858R_Sense_15b alone, were performed. The real-time ORNi-PCR and conventional real-time PCR were performed in the same manner as in Experimental Example 5-2, except for the PCR conditions. Table 14 shows the PCR conditions.

**[Table 14]**

| Temperature | Time | Cycle |
|---|---|---|
| 95°C | 1 min | 1 |
| 95°C | 15 sec | 45 |
| 57.5°C | 30 sec | |

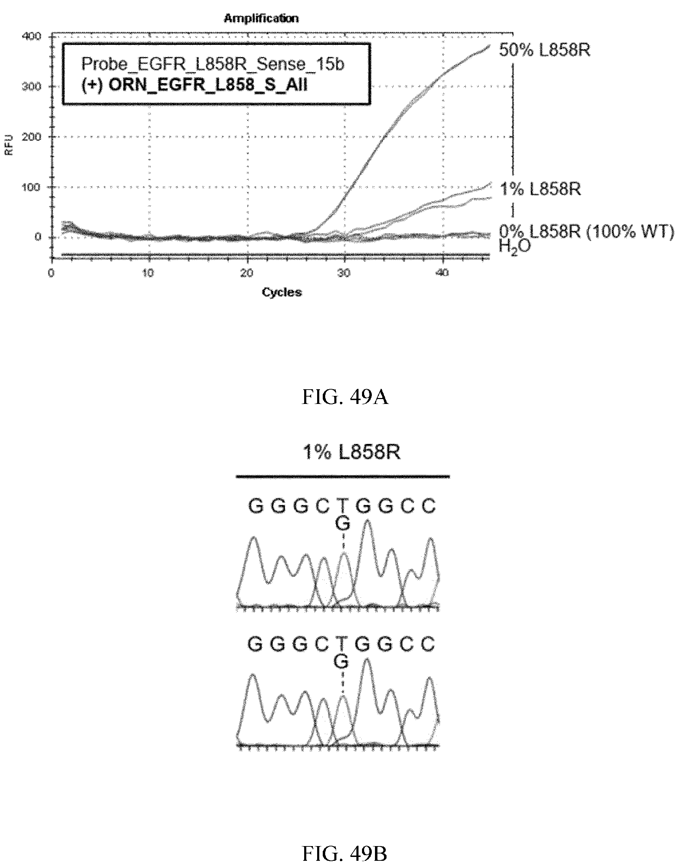

In real-time ORNi-PCR based on the combination of ORN_EGFR_L858_S_All and Probe_EGFR_L858R_Sense_15b, significant amplification was observed when the L858R EGFR mutation accounted for 1% of the total EGFR template (Figs. 49A and 49B).

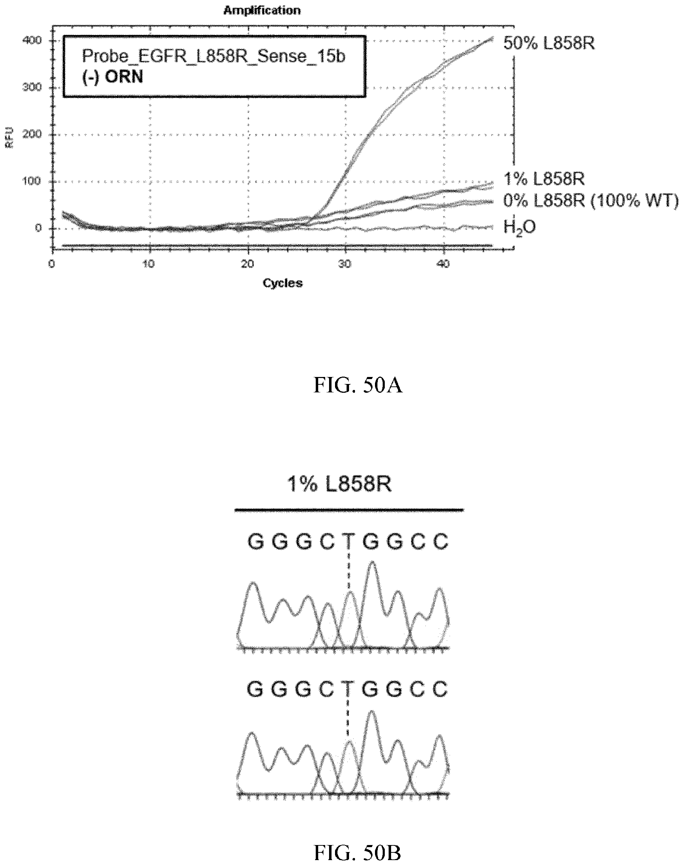

In contrast, conventional real-time PCR with Probe_EGFR_L858R_Sense_15b failed to detect mutations at the same WT-to-mutation ratio (Figs. 50A and 50B).

Thus, the phosphorothioate ORN was compatible with the conventional Taq DNA polymerase for real-time ORNi-PCR and increased specificity for detection of the target mutation.

### Experimental Example 5-4

GeneAce Taq DNA polymerase was also tested in real-time ORNi-PCR with phosphorothioate ORNs. ORNi-PCR was performed in the same manner as in Experimental Example 2-4, except for the use of GeneAce Taq DNA polymerase and PCR conditions. Table 15 shows the PCR conditions.

**[Table 15]**

| Temperature | Time | Cycle |
|---|---|---|
| 95°C | 10 min | 1 |
| 95°C | 20 sec | 40 |
| 59°C | 50 sec | |

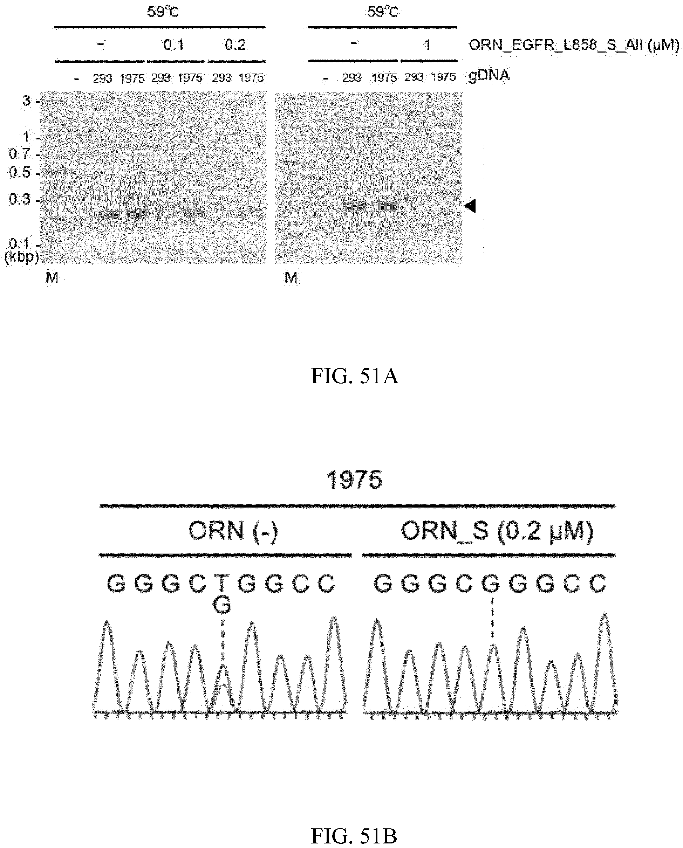

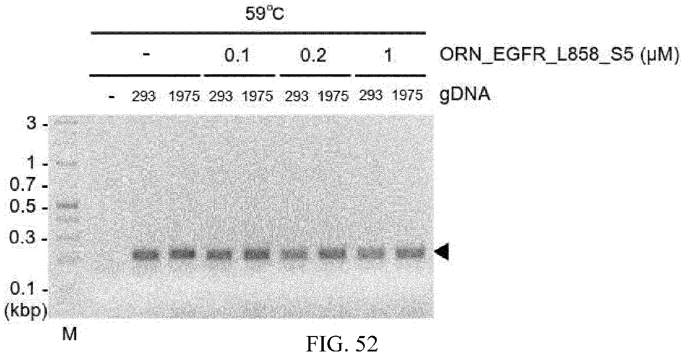

As shown in Figs. 51A and 51B, the fully phosphorothioated ORNs blocked target DNA amplification at lower concentrations (0.1 to 0.2 µM) than the unmodified ORNs, while higher concentrations (1 µM) resulted in non-specific inhibition of DNA amplification. The partially modified ORN (ORN_EGFR_L858_S_5) did not inhibit DNA elongation (Fig. 52).

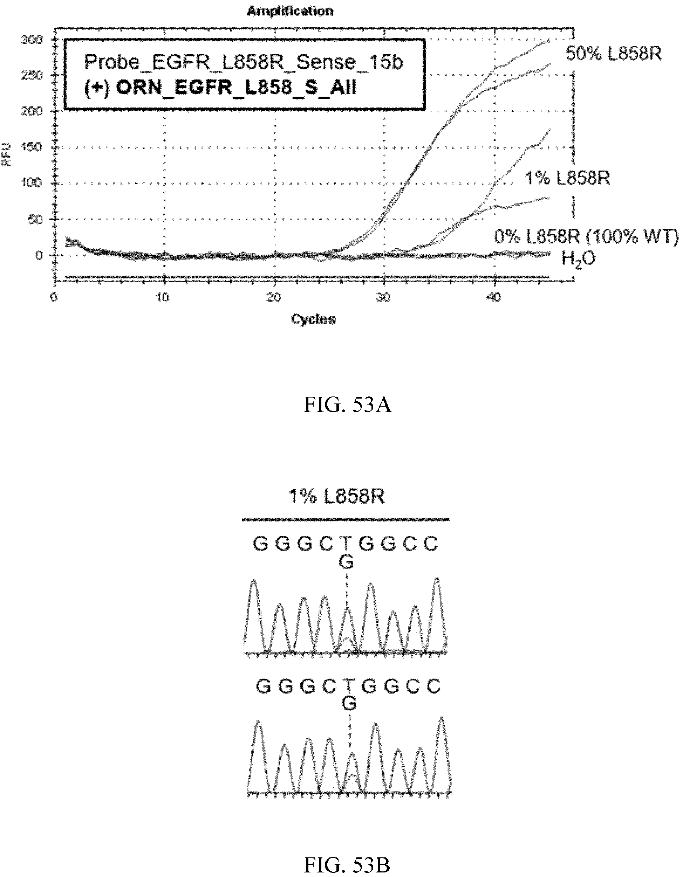

In real-time ORNi-PCR, strong amplification was observed when the L858R EGFR mutation accounted for 1% of the total EGFR template (Figs. 53A and 53B).

### Conclusion of Example 5

Thus, the phosphorothioated ORNs can also effectively block DNA elongation by the chemically modified Taq DNA polymerase.

### Example 6

### One-Step Real-Time Reverse Transcription-ORNi-PCR (RT-ORNi-PCR) Method Using Phosphorothioated ORNs

### Experimental Example 6-1

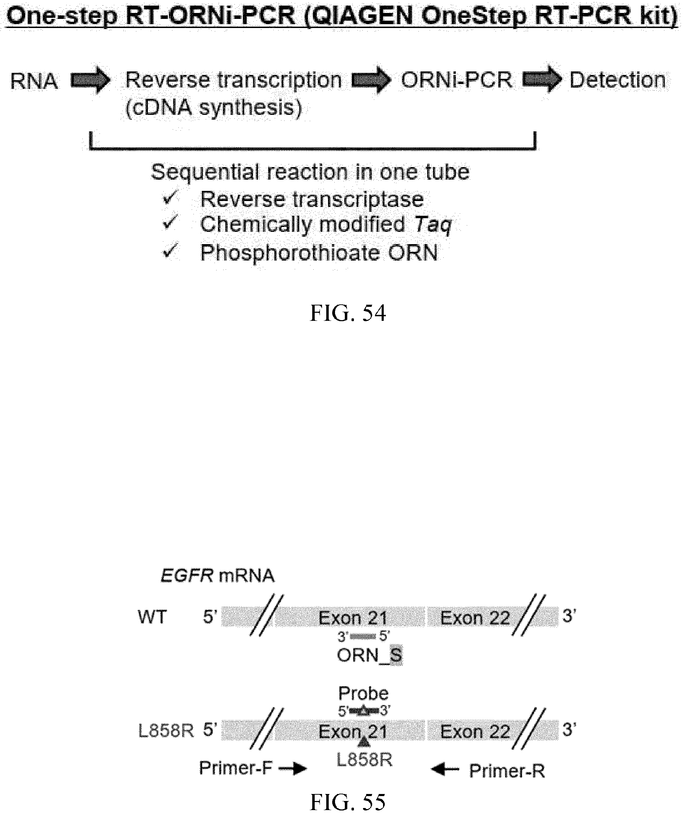

To detect single nucleotide mutations in RNA, one-step RT-ORNi-PCR was attempted using a one-step RT-PCR reagent (QIAGEN OneStep RT-PCR Kit) containing reverse transcriptase and chemically modified Taq DNA polymerase. Fig. 54 schematically shows a scheme of the one-step RT-ORNi-PCR. Fig. 55 schematically shows the location of mutations on the WT EGFR gene, ORN, probe and pair of primers.

ORN_EGFR_L858_S_All was used as the fully phosphorothioated ORN, and ORN_EGFR_L858 was used as the unmodified ORN. The pair of primers used includes hEGFR-Exon21-F8 (SEQ ID NO: 9) as the forward primer and hEGFR-T790M_L858R_cDNA-R5 (SEQ ID NO: 19) as the reverse primer. Table 16 shows the PCR conditions.

**[Table 16]**

| Temperature | Time | Cycle |
|---|---|---|
| 50°C | 30 min | 1 |
| 95°C | 15 min | 1 |
| 94°C | 30 sec | 30 |
| 57.5°C | 30 sec | |

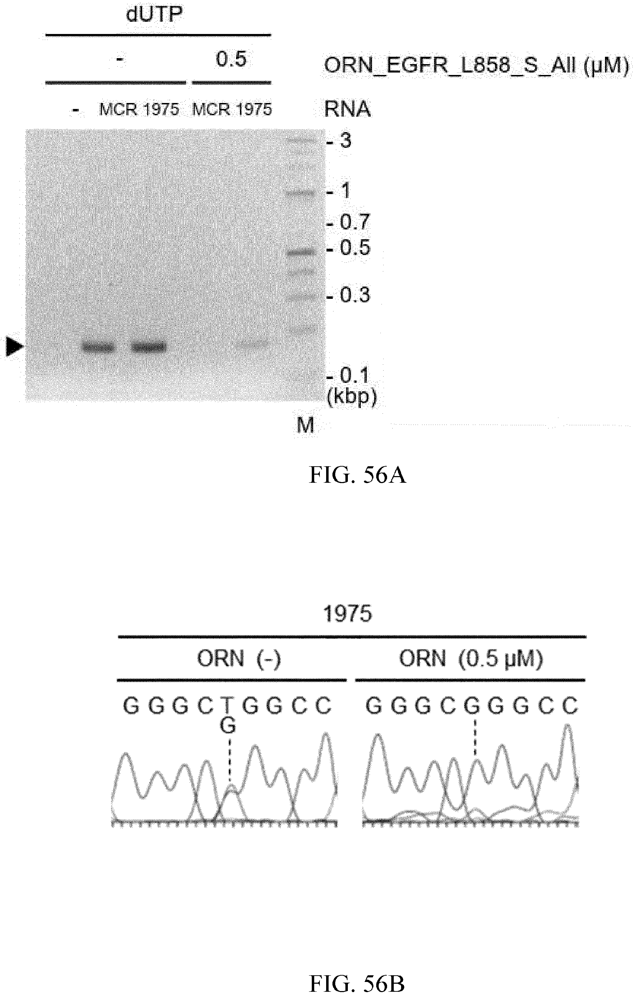

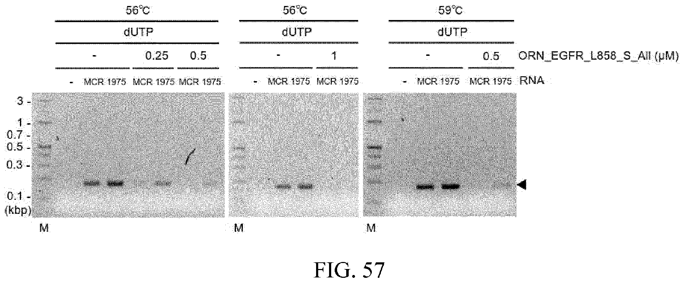

Results are shown in Figs. 56A, 56B and 57. One-step RT-ORNi-PCR using ORN_EGFR_L858_S_All (0.5 µM ORN; during the annealing and elongation steps at 57.5°C) successfully detected the L858R EGFR mutation using total RNA extracted from each of NCI-H1975 cells and MRC-5 cells with WT EGFR.

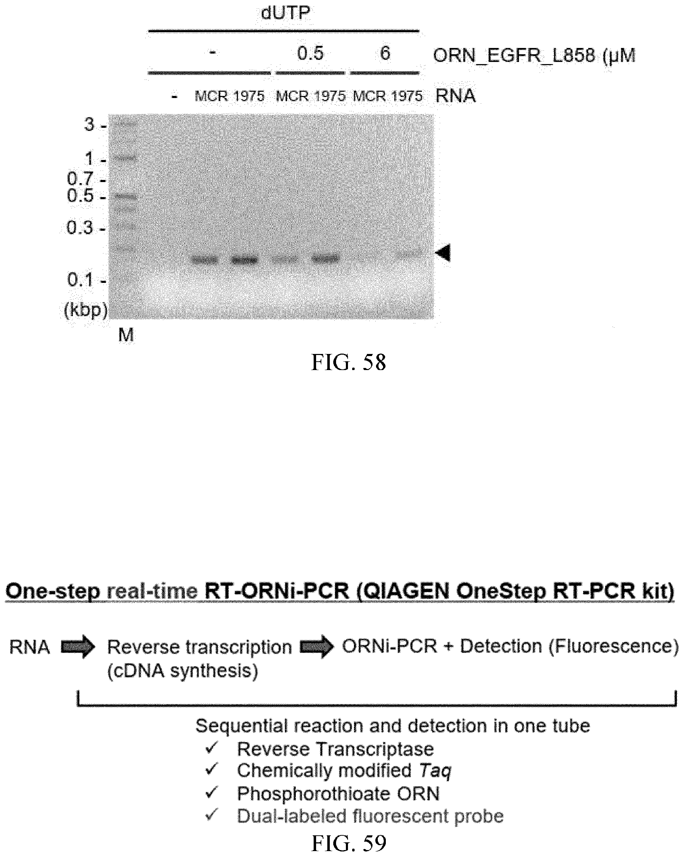

ORN_EGFR_L858, which is the unmodified ORN, did not show complete inhibition of target DNA amplification even at a higher concentration (6 µM) (Fig. 58). This suggested that the unmodified ORN may be degraded by reverse transcriptase and DNA polymerase in this reagent.

Thus, one-step RT-ORNi-PCR with the phosphorothioated ORNs was able to identify single nucleotide variations in RNA.

### Experimental Example 6-2

Although the QIAGEN OneStep RT-PCR Kit is not designed for real-time detection of DNA amplification, one-step real-time RT-ORNi-PCR was attempted using the QIAGEN OneStep RT-PCR Kit and double-labeled fluorescent probes. Fig. 59 schematically shows the scheme of one-step real-time RT-ORNi-PCR.

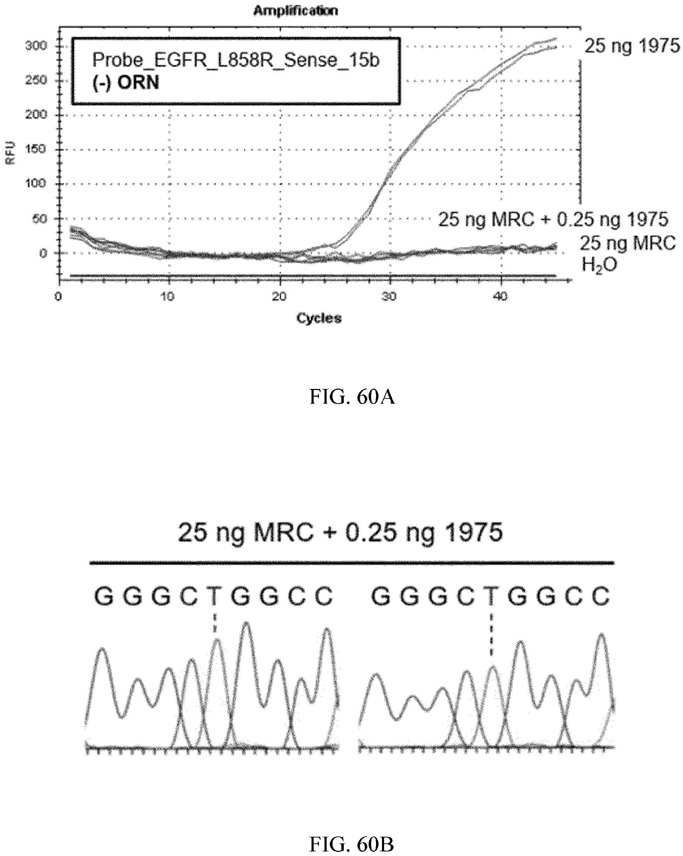

The L858R EGFR mutation in EGFR mRNA of NCI-H1975 cells was clearly detected by Probe_EGFR_L858R_Sense_15b (Fig. 60A); real-time detection was possible using the combination of this reagent (QIAGEN OneStep RT-PCR Kit) and the probe.

However, one-step real-time RT-PCR failed to detect the L858R EGFR mutation in mixed RNA, in which total MRC-5 RNA (25 ng) was mixed with NCI-H1975 (0.25 ng) so that NCI-H1975 RNA accounted for 1% of total RNA (Figs. 60A and 60B).

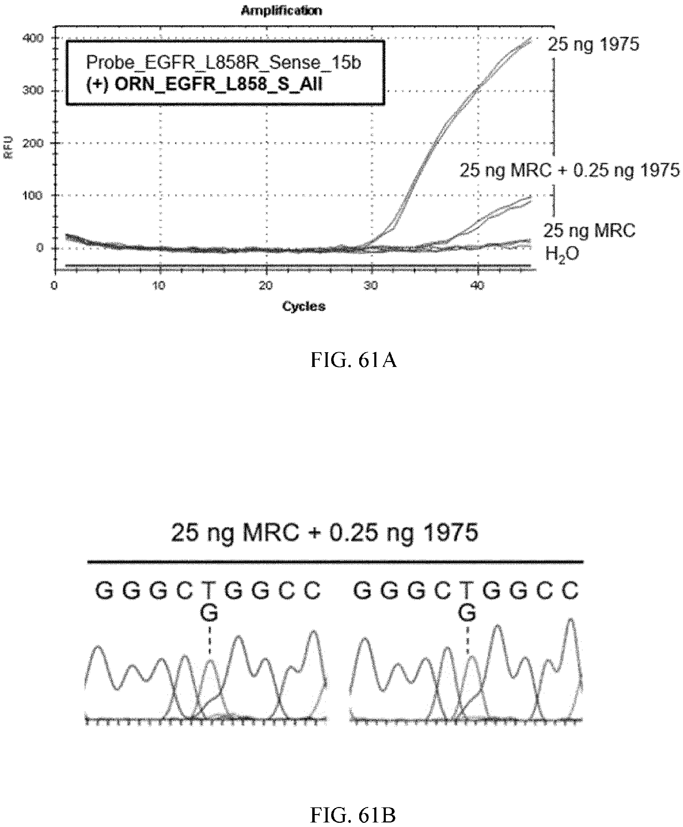

In contrast, one-step real-time RT-ORNi-PCR successfully detected the L858R EGFR mutation in the mixed total RNA (Figs. 61A and 61B), demonstrating a marked technical advantage in the detection of single-nucleotide differences in RNA.

### Experimental Example 6-3

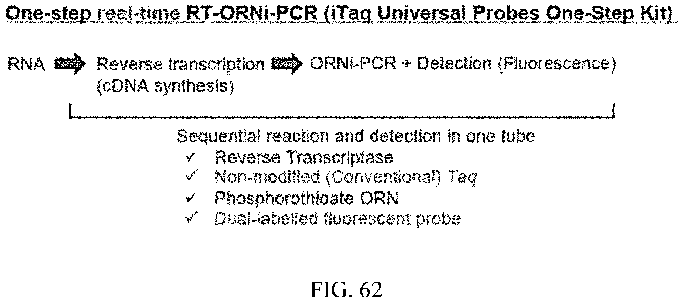

Next, the iTaq Universal Probes One-Step Kit containing unmodified Taq DNA polymerase was used to confirm the usefulness of one-step real-time RT-ORNi-PCR. Fig. 62 schematically shows the scheme of one-step real-time RT-ORNi-PCR using the iTaq Universal Probes One-Step Kit.

ORN_EGFR_L858_S_All was used as the fully phosphorothioated ORN. The pair of primers used was hEGFR-Exon21-F8 (SEQ ID NO: 9) as the forward primer and hEGFR-T790M_L858R_cDNA-R5 (SEQ ID NO:19) as the reverse primer. Table 17 shows the PCR conditions.

**[Table 17]**

| Temperature | Time | Cycle |
|---|---|---|
| 50°C | 10 min | 1 |
| 95°C | 2 min | 1 |
| 95°C | 10 sec | 45 |
| 57.5°C | 30 sec | |

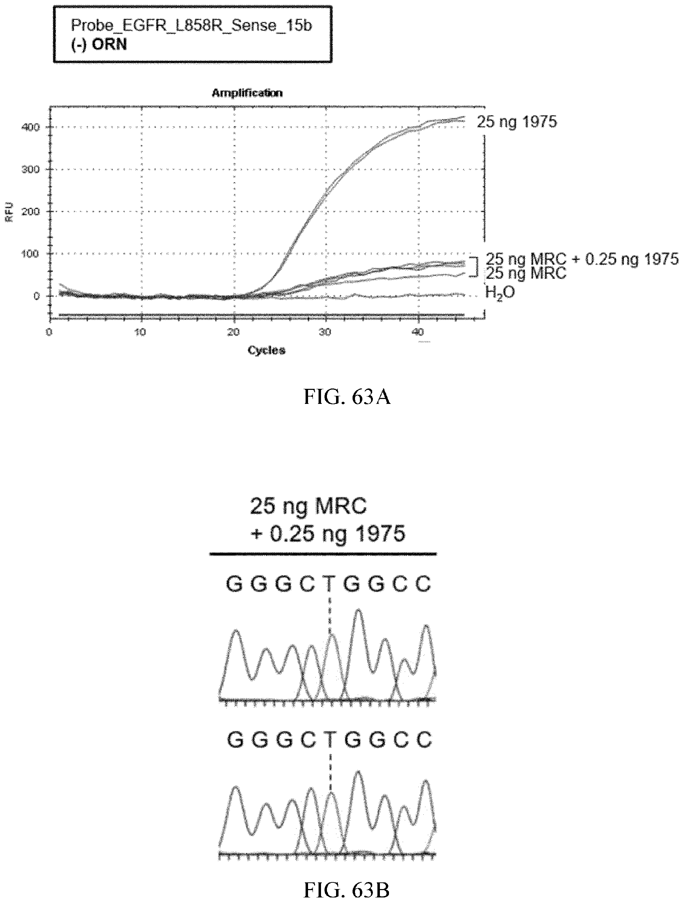

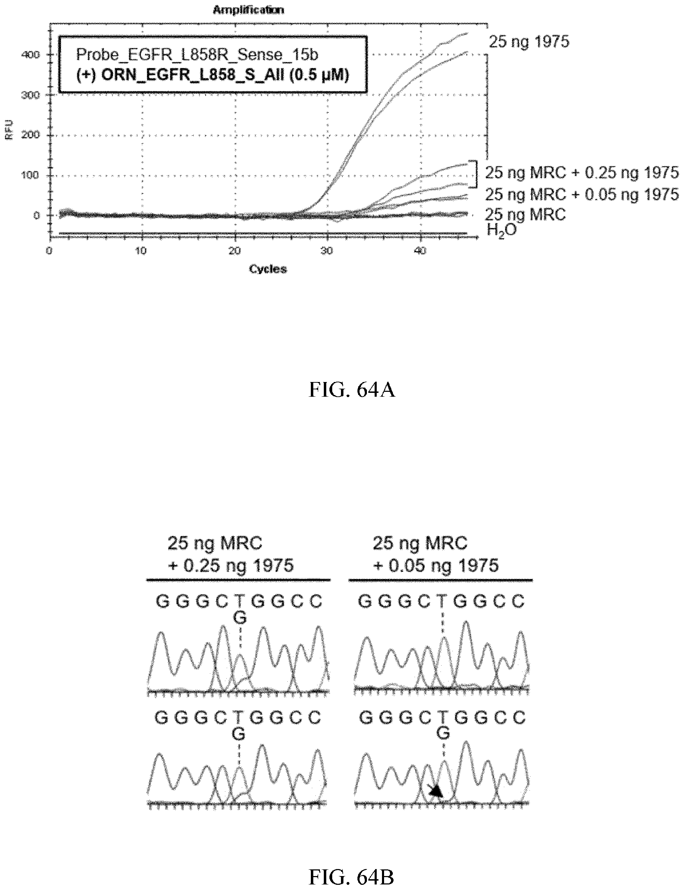

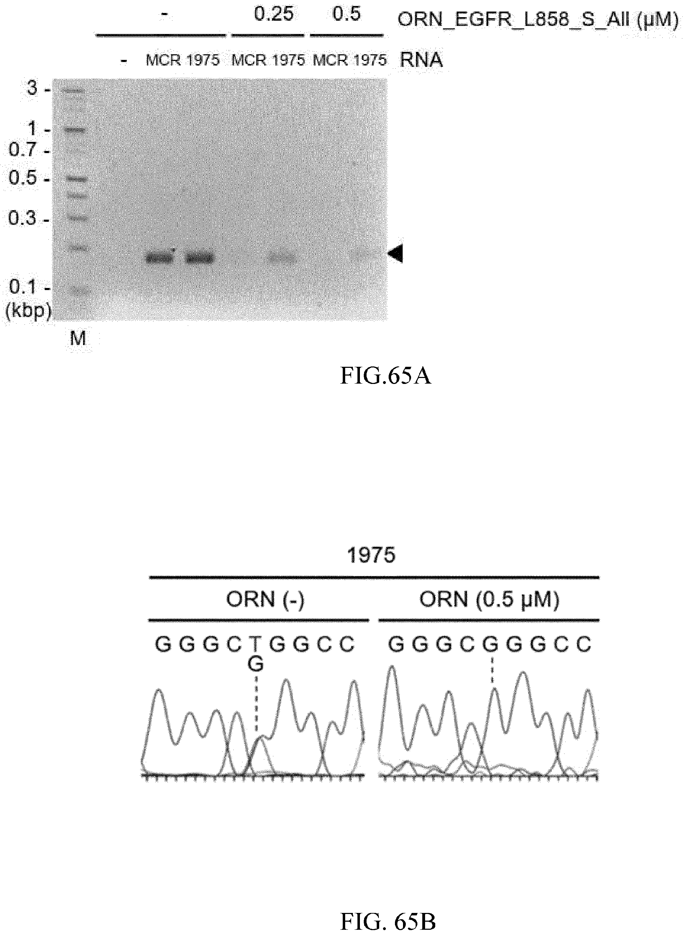

Unlike one-step real-time RT-PCR (Figs. 63A and 63B), one-step real-time RT-ORNi-PCR successfully detected the L858R EGFR mutation under optimized experimental conditions even when NCI-H1975 RNA was present at 1 to 0.2% in the total RNA (Figs. 64A, 64B, 65A and 65B).

Thus, one-step real-time RT-ORNi-PCR is superior to the conventional one-step real-time RT-PCR in terms of sensitivity and specificity in detecting single nucleotide variations in RNA.

### Experimental Example 6-4

When using 25 ng of NCI-H1975 total RNA, one-step real-time ORNi-RT-PCR showed lower quantitative cycles (Cq) than one-step real-time RT-PCR (compare Figs. 61A and 60A, and Figs. 64 and 63).

Since ORN_EGFR_L858R_S_All is complementary to EGFR mRNA (Fig. 55), it was assumed that it also binds to the mRNA during the RT step of the one-step RT-ORNi-PCR reaction, thereby suppressing cDNA synthesis by reverse transcriptase.

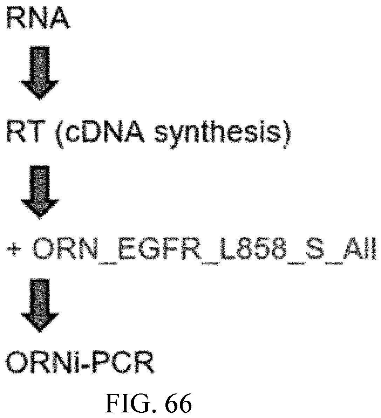

Therefore, the stage at which this ORN inhibits DNA elongation in the one-step RT-ORNi-PCR reaction in a sequence-specific manner was investigated. Fig. 66 schematically illustrates the steps of this investigation. RT-ORNi-PCR was performed in the same manner as in Experimental Example 6-3, except for the timing of ORN_EGFR_L858R_S_All addition.

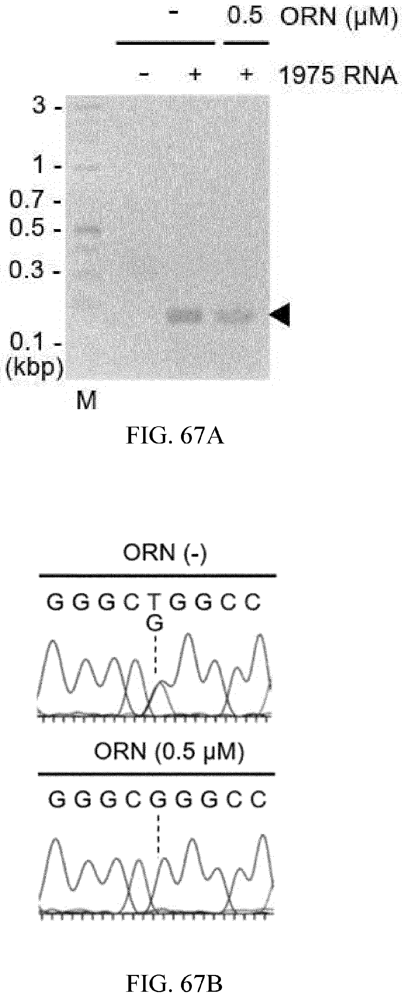

As shown in Figs. 67A and 67B, this ORN suppressed amplification of target DNA from cDNA in a sequence-specific manner during the PCR step.

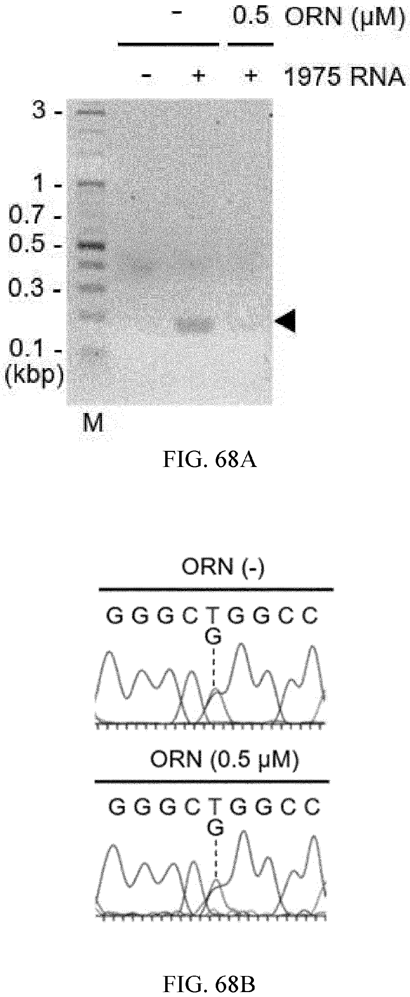

When RT was performed in the presence of this ORN, followed by purification of the cDNA and subsequent PCR, DNA amplification was nonspecifically suppressed (Figs. 68A and 68B).

### Experimental Example 6-5

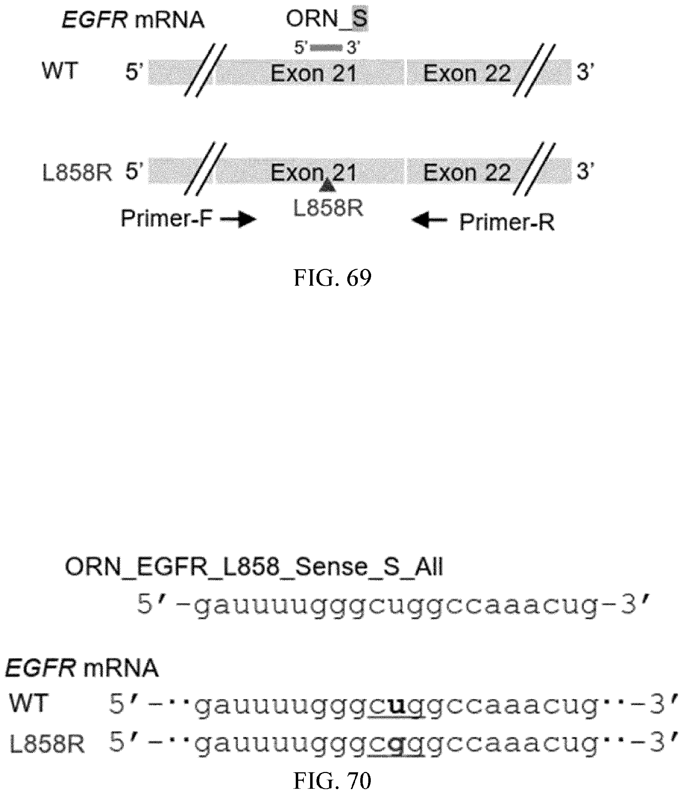

ORN_EGFR_L858_Sense_S_All (SEQ ID NO: 18), which is a phosphorothioated ORN noncomplementary to EGFR mRNA, was designed (Figs. 69 and 70). One-step RT-ORNi-PCR was performed in the same manner as in Experimental Example 6-3, except for the use of ORN_EGFR_L858_Sense_S_All and PCR conditions. Table 18 shows the PCR conditions.

**[Table 18]**

| Temperature | Time | Cycle |
|---|---|---|
| 50°C | 10 min | 1 |
| 95°C | 2 min | 1 |
| 95°C | 10 sec | 30 |
| 57.5°C | 30 sec | |

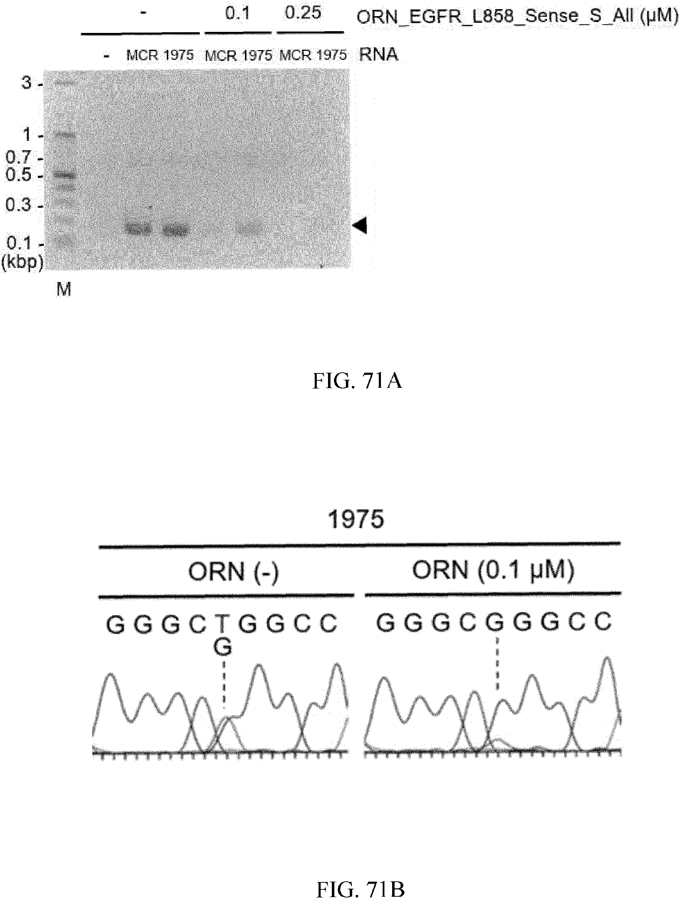

As shown in Figs. 71A and 71B, ORN_EGFR_L858_Sense_S_All also suppressed DNA synthesis of WT EGFR in the one-step RT-ORNi-PCR reaction.

### Experimental Example 6-6

Therefore, the stage at which this ORN inhibits DNA elongation in the one-step RT-ORNi-PCR reaction in a sequence-specific manner was investigated. The steps of this investigation were the same as those shown in Fig. 66, except for the use of ORN_EGFR_L858_Sense_S_All. In this investigation, RT-ORNi-PCR was performed in the same manner as in Experimental Example 6-5, except for the timing of ORN addition.

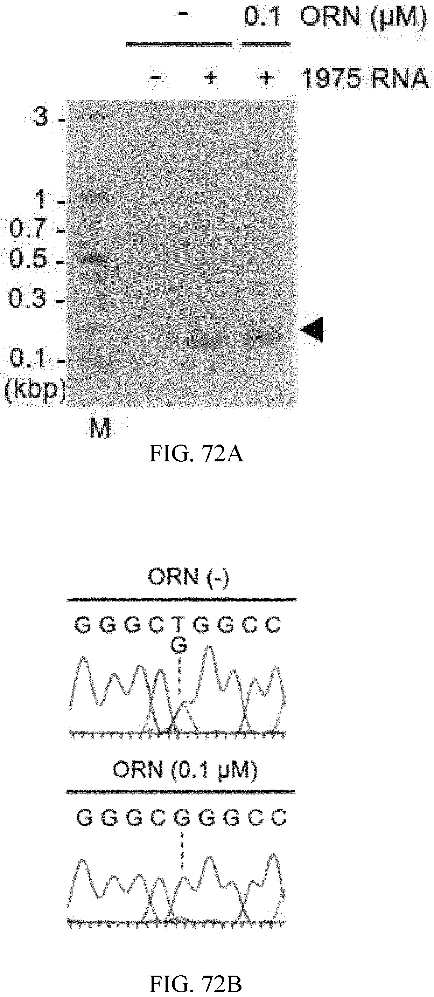

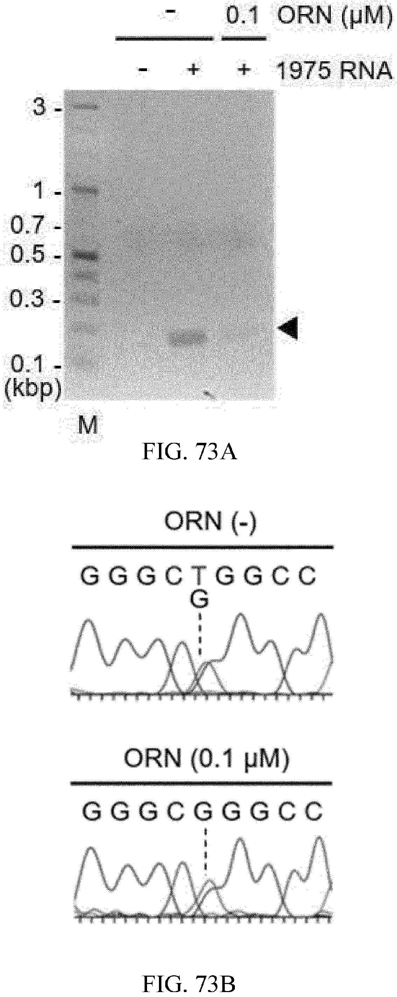

As shown in Figs. 72A and 72B, this ORN suppressed amplification of target DNA from cDNA in a sequence-specific manner during the PCR step. When RT was performed in the presence of this ORN, followed by cDNA purification and subsequent PCR, DNA amplification was nonspecifically suppressed, indicating that this ORN caused nonspecific inhibition during the RT step (Figs. 73A and 73B).

Thus, single-nucleotide differences are discriminated during the PCR step of one-step RT-ORNi-PCR. Although it was found that phosphorothioated ORNs nonspecifically inhibit reverse transcriptase activity, this inhibition was shown not to affect single-nucleotide discrimination in one-step RT-ORNi-PCR.

Therefore, both complementary and non-complementary phosphorothioate ORNs to the target RNA can be used for one-step RT-ORNi-PCR without limiting the freedom of ORN design.

## Claims

1. A kit comprising:
a DNA polymerase usable in PCR; and,
a single-stranded RNA,
wherein either (1) the DNA polymerase is subjected to a temporary inactivation treatment, or (2) the single-stranded RNA is resistant to degradation by RNA degrading enzymes, or both (1) and (2),
the DNA polymerase comprises 5' to 3' exonuclease activity, and
the single-stranded RNA comprises a nucleotide sequence capable of hybridizing to a target sequence in a target nucleic acid molecule.

2. The kit according to claim 1, further comprising a nucleic acid probe comprising a fluorescent dye label,
wherein a nucleotide sequence of the nucleotide probe differs from the nucleotide sequence of the single-stranded RNA by comprising at least one mutation.

3. The kit according to claim 1, wherein the single-stranded RNA resistant to the degradation by the RNA degrading enzymes is a phosphorothioate type single-stranded RNA.

4. The kit according to claim 1, wherein the DNA polymerase subjected to a temporary inactivation treatment is a DNA polymerase modified by a dicarboxylic anhydride.

5. The kit according to claim 1, further comprising dUTP.

6. A method for specifically inhibiting nucleic acid amplification of a target region, comprising:
a provision step of providing a mixture comprising a DNA polymerase usable in PCR, a single-stranded RNA, a target nucleic acid molecule, a pair of DNA primers, and dNTPs;
a denaturation step of maintaining the mixture at a denaturation temperature or higher;
an annealing step of maintaining the mixture at annealing temperature;
a DNA elongation step of maintaining the mixture at a DNA elongation temperature;
an amplification step of amplifying DNA strands by repeating, in order, the denaturation step, the annealing step and the DNA elongation step; and
a detection step of detecting amplification products obtained by the amplification of the DNA strands,
wherein either (1) the DNA polymerase is temporarily inactivated, or (2) the single-stranded RNA is resistant to degradation by RNA degrading enzymes, or both (1) and (2),
the DNA polymerase comprises 5' to 3' exonuclease activity, and
the single-stranded RNA comprises a nucleotide sequence capable of hybridizing to a target sequence in the target nucleic acid molecule.

7. The method according to claim 6, wherein the mixture further comprises a nucleic acid probe comprising a fluorescent dye label,
a nucleotide sequence of the nucleic acid probe differs from the nucleotide sequence of the single-stranded RNA by comprising at least one mutation, and
the amplification products are detected by fluorescence of the fluorescent dye label.

8. The method according to claim 6, wherein the single-stranded RNA resistant to the degradation by the RNA degrading enzymes is a phosphorothioate type single-stranded RNA.

9. The method according to claim 6, wherein the DNA polymerase subjected to the temporary inactivation treatment is a DNA polymerase modified by a dicarboxylic anhydride.

10. The method according to claim 9, wherein the dNTPs comprise dATP, dUTP, dGTP, and dCTP, and optionally deoxythymidine triphosphate (dTTP) at a concentration lower than the dUTP.

11. The method according to claim 6, further comprising a reverse transcription step of obtaining the target nucleic acid molecule from a target RNA using a reverse transcriptase.

12. A method for specifically inhibiting nucleic acid amplification of a target region, comprising:
a provision step of providing a mixture comprising a DNA polymerase usable in PCR, a single-stranded RNA, a target nucleic acid molecule, a DNA primer, and dNTPs;
a denaturation step of maintaining the mixture at a denaturation temperature or higher;
a maintenance step of maintaining the mixture at a temperature in the range of 45°C to 54°C;
an amplification step of amplifying DNA strands by repeating, in order, the denaturation step and the maintenance step; and
a detection step of detecting amplification products obtained by the amplification of the DNA strands,
wherein the DNA polymerase comprises 5' to 3' exonuclease activity, and
the single-stranded RNA comprises a nucleotide sequence capable of hybridizing to a target sequence in the target nucleic acid molecule.

13. The method according to claim 12, wherein the mixture further comprises a nucleic acid probe comprising a fluorescent dye label,
a nucleotide sequence of the nucleic acid probe differs from the nucleotide sequence of the single-stranded RNA by comprising at least one mutation, and
the amplification products are detected by fluorescence of the fluorescent dye label.

14. The method according to claim 12, further comprising a reverse transcription step of obtaining the target nucleic acid molecule from a target RNA using a reverse transcriptase.
